# EUROPEAN PATENT APPLICATION

(11) **EP 4 397 320 A1**
(43) Date of publication of application: **10.07.2024**
(21) Application number: 22864604.8
(22) Date of filing: 31.08.2022
(51) Int. Cl.: A61K 47/68, A61K 47/42, A61K 51/10, A61P 35/00, C07D 403/12, C07K 16/00

(54) **RADIOACTIVE COMPLEX OF DEGLYCOSYLATED ANTIBODY AND RADIOACTIVE PHARMACEUTICAL PRODUCT**

(30) Priority: 31.08.2021 JP 2021141625
(71) Applicant: Nihon Medi-Physics Co., Ltd., Tokyo 136-0075 (JP)
(72) Inventor: KISHIMOTO, Satoshi, Tokyo 136-0075 (JP); TAKEDA, Takuya, Tokyo 136-0075 (JP); NARITA, Yudai, Tokyo 136-0075 (JP); KAWATANI, Minoru, Tokyo 136-0075 (JP); IZAWA, Akihiro, Tokyo 136-0075 (JP); TARUMIZU, Yuta, Tokyo 136-0075 (JP)
(74) Representative: J A Kemp LLP
(86) International application number: PCT/JP2022/032709
(87) International publication number: WO 2023/033022

(57) **Abstract**

The present invention provides "a conjugate of a chelating agent chelated with a radionuclide and a deglycosylated antibody, wherein the chelating agent site-specifically modifies the Fc region of the aforementioned antibody via a linker". The conjugate is improved in the risk of a decrease in the effect and the risk of side effects.

## Description

### [Technical Field]

The present invention relates to a conjugate of a deglycosylated antibody and a radionuclide, a radiopharmaceutical containing same, and a production method thereof.

### [Background Art]

Antibodies have conventionally been widely used to detect target molecules in various researches and developments, and have become extremely important in industry as detection reagents and diagnostic agents. Also, antibodies are attracting attention as pharmaceutical products for treating diseases in view of their high specificity for the target molecules.

Antibodies have the property of accumulating in reticuloendothelial system tissue through interaction with FcγR expressed by immune cells. For the purpose of improving the pharmacokinetics of antibodies, therefore, techniques to radiolabel antibodies with cleaved sugar chains (hereinafter to be also simply referred to as deglycosylated antibodies) have been reported (Non Patent Literature 1). Also, Patent Literature 1 discloses PET experiments with radiolabeled (⁸⁹Zr) deglycosylated antibodies.

On the other hand, as a site-specific chemical modification to add function without affecting the specificity of the antibody to the target molecule, antibody-modification peptide (to be also referred to as CCAP (Chemical conjugation by affinity peptide) peptide) that binds specifically or selectively to antibodies has been reported (Patent Literature 2). Furthermore, the present Applicant has reported on techniques for introducing an amino acid capable of binding to a crosslinking agent into a CCAP peptide, and modifying antibodies with labeling substances such as a complex containing a radioisotope bonded to a crosslinking agent and medicaments such as anticancer agent and the like via the bond between the amino acid and the crosslinking agent (Patent Literatures 3, 4).

### [Citation List]

### [Patent Literature]

[Patent Literature 1]
   US-A-2019/0389966 specification
[Patent Literature 2]
   WO 2016/186206
[Patent Literature 3]
   WO 2017/217347
[Patent Literature 4]
   WO 2021/075546

### [Non Patent Literature]

[Non Patent Literature 1]
Vivier D. et al., J Nucl Med. 2019 Aug; 60(8):1174-1182.

### [Summary of Invention]

### [Technical Problem]

With RI-labeled antibodies, accumulation in normal tissues and decrease in tumor accumulation become the problem.

For ⁸⁹Zr-labeled deglycosylated antibodies in Non Patent Literature 1 and Patent Literature 1, control of the number of modifications becomes the problem. Also, radiation therapy with deglycosylated antibodies has not been noted.

In addition, whether deglycosylated antibodies can be modified with CCAP peptide is not known.

Therefore, provision of RI-labeled antibodies that reduce accumulation in normal tissues and improve tumor accumulation becomes the task.

### [Solution to Problem]

One embodiment of the present invention is a conjugate of a deglycosylated antibody and a chelating agent, wherein the chelating agent is chelated with a metal radionuclide, and the chelating agent site-specifically modifies the Fc region of the aforementioned antibody via linker (L). Another embodiment of the present invention is a conjugate of a deglycosylated antibody and a chelating agent, wherein the chelating agent is chelated with a metal radionuclide, and the metal radionuclide is an α-ray emitting nuclide.

Another embodiment of the present invention is a method for producing the above-mentioned conjugate.

Another embodiment of the present invention is a radiopharmaceutical containing the above-mentioned conjugate as an active ingredient.

The "linking" in the present invention means both direct connection and indirect connection, unless otherwise specified.

### [Advantageous Effects of Invention]

According to the present invention, an RI-labeled antibody that shows decreased accumulation in normal tissue and improved accumulation in tumor is provided.

### [Brief Description of Drawings]

[Fig. 1]
   A graph showing the evaluation results of the binding activity of the radioconjugates produced and formulated according to the descriptions in Example 1 (PNGase F-treated Trastuzumab) and Comparative Example 1 (Trastuzumab) to HER2.
[Fig. 2]
   A graph showing the results of confirmed distribution of the radioconjugates produced and formulated according to the descriptions in Example 1 (⁸⁹Zr-CCAP-Trastuzumab-PNGaseF) and Comparative Example 1 (⁸⁹Zr-CCAP-Trastuzumab) to each tissue in the living body (blood, heart, lung, liver, spleen, pancreas, stomach, small intestine, large intestine, kidney, bladder, thigh muscle, femur, skin, tumor, remained whole body).
[Fig. 3]
   Graphs showing the results of confirmed distribution of the radioconjugates produced and formulated according to the descriptions in Example 1 (⁸⁹Zr-CCAP-Trastuzumab-PNGaseF) and Comparative Example 1 (⁸⁹Zr-CCAP-Trastuzumab) to each tissue in the living body (blood, liver, spleen, tumor).
[Fig. 4]
   A graph showing the evaluation results of the antigen binding activity of the radioconjugates produced according to the descriptions in Example 2 (PNGase F-treated Trastuzumab) and Comparative Example 2 (Trastuzumab).
[Fig. 5]
   A graph showing the time-course profile of the tumor volume of the tumor-bearing mice of radioconjugate (Example 2) 10 kBq administration group (²²⁵Ac-Trastuzumab-PNGaseF_10KBq), radioconjugate (Example 2) 5 kBq administration group (²²⁵Ac-Trastuzumab-PNGaseF_5KBq), radioconjugate (Comparative Example 2) 10 kBq administration group (²²⁵Ac-Trastuzumab_10KBq), radioconjugate (Comparative Example 2) 5 kBq administration group (²²⁵Ac-Trastuzumab_5KBq), and Vehicle group.
[Fig. 6]
   A graph showing the time-course profile of the body weight of the tumor-bearing mice of radioconjugate (Example 2) 10 kBq administration group (²²⁵Ac-Tmab-PNG._10KBq), radioconjugate (Example 2) 5 kBq administration group (²²⁵Ac-Tmab-PNG._5KBq), radioconjugate (Comparative Example 2) 10 kBq administration group (²²⁵Ac-Tmab-Cont._10KBq), radioconjugate (Comparative Example 2) 5 kBq administration group (²²⁵Ac-Tmab-Cont._5KBq), and Vehicle group.
[Fig. 7]
   A graph showing the time-course number of surviving individuals in the tumor-bearing mice of radioconjugate (Example 2) 10 kBq administration group (²²⁵Ac-Trastuzumab-PNGaseF_10KBq), radioconjugate (Example 2) 5 kBq administration group (²²⁵Ac-Trastuzumab-PNGaseF_5KBq), radioconjugate (Comparative Example 2) 10 kBq administration group (²²⁵Ac-Trastuzumab_10KBq), radioconjugate (Comparative Example 2) 5 kBq administration group (²²⁵Ac-Trastuzumab_5KBq), and Vehicle group.
[Fig. 8]
   A graph showing the evaluation results of the antigen binding activity of the radioconjugates produced according to the descriptions in Example 3 (PNGase F-treated Cetuximab) and Comparative Example 3 (Cetuximab).
[Fig. 9]
   A graph showing the results of confirmed distribution of the radioconjugates produced according to the descriptions in Example 3 (⁸⁹Zr-CCAP-Cetuximab-PNGaseF) and Comparative Example 3 (⁸⁹Zr-CCAP-Cetuximab) to each tissue in the living body (blood, heart, lung, spleen, pancreas, stomach, small intestine, large intestine, thigh muscle (muscle), femur (bone), skin, tumor, kidney, liver, remained whole body).
[Fig. 10]
   A graph showing the results of confirmed distribution of the radioconjugates produced according to the descriptions in Example 3 and Comparative Example 3 to each tissue in the living body (blood, liver, spleen, tumor).

### [Description of Embodiments]

### (1) Radioconjugate 1

The present invention provides a conjugate of a chelating agent chelated with a metal radionuclide, and a deglycosylated antibody, in which the chelating agent site-specifically modifies the Fc region of the aforementioned antibody via a linker (hereinafter also to be referred to as the radioconjugate or simply conjugate of the present invention).

### (1-1) Metal radionuclide

The metal radionuclide contained in the radioconjugate of the present invention is a radionuclide that emits α-ray, a radionuclide that emits β-ray, a radionuclide that emits positron, or a radionuclide that emits γ-ray. When the radioconjugate of the present invention is used for cancer therapy, it is preferable to use a radionuclide that emits α-ray or a radionuclide that emits β-ray. When the radioconjugate of the present invention is used for cancer diagnosis or detection, it is preferable to use a radionuclide that emits positron, or a radionuclide that emits γ-ray. Examples of the radionuclide that emits α-ray include Bi-212, Bi-213, Ac-225, and Th-227. Examples of the radionuclide that emits β-ray include Co-60, Fe-59, Cu-64, Cu-67, Sr-89, Y-90, Tc-99 m, Ru-103, Sm-153, Dy-165, Ho-166, Lu-177, Re-186, Re-188, Au-198, Hg-203, Bi-212, Bi-213, and Pb-212. Examples of the radionuclide that emits positron include Cu-64, Ga-68, Y-86, and Zr-89. Examples of the radionuclide that emits γ-ray include Tc-99m and In-111. The metal radionuclide to be contained in the radioconjugate 1 of the present invention is more preferably Ac-225, Y-90, Lu-177, or Zr-89. The metal radionuclide contained in the radioconjugate 1 of the present invention is particularly preferably Ac-225 or Zr-89.

### (1-2) Antibody

The antibody contained in the conjugate of the present invention is not particularly limited as long as it is an antibody with cleaved sugar chain, namely, a deglycosylated antibody. The antibody to be used in the present invention may be a polyclonal antibody or a monoclonal antibody, preferably a monoclonal antibody. The origin of the antibody is not particularly limited, and examples include antibodies of non-human animals, non-human mammals, and humans, preferably antibodies of human, rat, mouse, and rabbit. When the antibody is derived from species other than human, it is preferably chimerized or humanized using well-known techniques. The antibody to be used in the present invention may be a chimera antibody, a humanized antibody, or a human antibody. The antibody to be used in the present invention may be a bispecific antibody. More preferably, the deglycosylated antibody used in the complex of the present invention is human IgG antibody. The human IgG antibody may be, for example, IgG1, IgG2, IgG3, or IgG4.

Humanized antibody (IgG) has an N-linked sugar chain at the Asn residue (residue 297 in EU numbering) of the Fc region of the heavy chain. Furthermore, an N-linked sugar chain or an O-linked sugar chain may be bound to an amino acid residue other than Asn297. The deglycosylated antibody used in the present invention has these sugar chains cleaved, preferably has the N-linked sugar chain cleaved, and more preferably has the N-linked sugar chain of the Asn residue (residue 297) cleaved.

Antibodies can be deglycosylated by methods generally used in the field, and can be chemically or enzymatically deglycosylated. For chemical deglycosylation, the descriptions of Hakimuddin et al., 1987, Arch. Biochem. Biophys. 259:52 and Edge et al., 1981, Anal. Biochem. 118:131 can be referred to. For enzymatic cleavage, the description of Thotakura et al., 1987, Meth. Enzymol. 138:350 can be referred to, and various sugar chain degrading enzymes can be used. The origin of the enzyme is not particularly limited, and it can be derived from plant, bacterium, or yeast. An enzyme that cleaves (releases) N-linked sugar chain is preferred, and peptide-N⁴-(N-acetyl-β-glucosaminyl)-asparagine amidase (PNGaseF, PNGaseA, etc.) classified as EC3.5.1.52, endoglycosidase classified as EC3.2.1.96, and the like can be mentioned. As the endoglycosidase, endo-β-N-acetylglucosaminidase D (endoglycosidase D, Endo-D, endo-D), endo-β-N-acetylglucosaminidase H (endoglycosidase H, Endo-H, endo-H), endoglycosidase S (EndoS, Endo-S, endo-S), endo-β-N-acetylglucosaminidase M (endoglycosidase M, Endo-M, endo-M), endo-β-N-acetylglucosaminidase LL (endoglycosidase LL, EndoLL, Endo-LL, endo-LL), endo-β-N-acetylglucosaminidase F1 (endoglycosidase F1, Endo-F1, endo-F1), endo-β-N-acetylglucosaminidase F2 (endoglycosidase F2, Endo-F2, endo-F2), and endo-β-N-acetylglucosaminidase F3 (endoglycosidase F3, Endo-F3, endo-F3) can be specifically mentioned.

Deglycosylation treatment (reaction temperature, reaction time, etc.) of antibodies can be performed according to methods practiced in the art, depending on the means used to cleave (release) sugar chains.

Specific preferred examples of the antibody used in the conjugate of the present invention include anti-HER2 antibody (e.g., trastuzumab, pertuzumab), anti-EGFR antibody (e.g., cetuximab, panitumumab), and anti-CD20 antibody (e.g., rituximab, ibritumomab, ocrelizumab).

All can be prepared by methods known per se or are commercially available.

The antibody used in the conjugate of the present invention may be the antibody described in WO 2017/141604 or WO 2021/075545.

### (1-3) Chelating agent

In the present invention, the chelating agent is not particularly limited as long as it has a site in the structure thereof where metal radionuclide is coordinated. Examples of the chelating agent include CB-TE2A (1,4,8,11-Tetraazabicyclo[6.6.2]hexadecane-4,11-diacetic acid), CDTA (Cyclohexane-trans-1,2-diamine tetra-acetic acid), CDTPA (4-cyano-4-[[(dodecylthio)thioxomethyl]thio]-pentanoic acid), DOTA (1,4,7,10-Tetraazacyclododecane-1,4,7,10-tetraacetic acid), DOTMA ((1R,4R,7R,10R)-α,α',α",α‴-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTPA (1,4,7,10-tetraazacyclododecane-1,4,7,10-tetra propionic acid), 1,4,7,10-tetrakis(pyridin-2-ylmethyl)-1,4,7,10-tetraazacyclododecane (L^{py}), DOTA-GA (α-(2-Carboxyethyl)-1,4,7,10-tetraazacyclododecane-1,4,7,10-tetraacetic acid), DOTP (((1,4,7,10-Tetraazacyclododecane-1,4,7,10-tetrayl)tetrakis(methylene))tetraphosphonic acid), DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), D02P (Tetraazacyclododecane dimethanephosphonic acid), Deferoxamine (DFO), DTPA (Glycine, N,N-bis[2-[bis(carboxymethyl)amino]ethyl]-), CHX-A"-DTPA (2,2'-((2-(((1S,2R)-2-(bis(carboxymethyl)amino)cyclohexyl) (carboxymethyl)amino)ethyl) azanediyl)diacetic acid), DTPA-BMA (5,8-Bis(carboxymethyl)-11-[2-(methylamino)-2-oxoethyl]-3-oxo-2,5,8,11-tetraazatridecan-13-oic acid), EDTA (2,2',2",2‴-(ethane-1,2-diylbis(azanetriyl))tetraacetic acid), NOTA (1,4,7-Triazacyclononane-1,4,7-triacetic acid), NOTP (1,4,7-Triazacyclononane-1,4,7-triyltris(methylenephosphonic acid)), TETPA (1,4,8,11-tetraazacyclotetradecane-1,4,8,11-tetrapropionic acid), TETA (1,4,8,11-Tetraazacyclotetradecane-N,N',N",N‴-tetraacetic acid), TTHA (3,6,9,12-Tetrakis(carboxymethyl)-3,6,9,12-tetraazatetradecanedioic acid), HEHA (1,2,7,10,13-hexaazacyclooctadecane-1,4,7,10,13,16-hexaacetic acid), 1,2-HOPO (N,N',N",N‴-tetra(1,2-dihydro-1-hydroxy-2-oxopyridine-6-carbonyl)-1,5,10,14-tetraazatetradecane), PEPA (1,4,7,10,13-pentaazacyclopentadecane-N,N',N",N‴,Nʺʺ-pentaacetic acid), H4octapa (N,N'-bis(6-carboxy-2-pyridylmethyl)-ethylenediamine-N,N'-diacetic acid), H2bispa2 (6,6'-({9-hydroxy-1,5-bis(methoxycarbonyl)-2,4-di(pyridin-2-yl)-3,7-diazabicyclo[3.3.1]nonane-3,7-diyl}bis(-methylene))dipicolinic acid), H2dedpa (1,2-[{6-(carboxy)-pyridin-2-yl}-methylamino]ethane), H2macropa (6-(1,4,10,13-tetraoxa-7,16-diazacyclooctadecan-N,N'-methyl)picolinic acid), H5decapa (N,N"-bis(6-carboxy-2-pyridylmethyl)-diethylenetriamine-N,N',N"-triacetic acid), H6phospa (N,N'-(methylenephosphonate)-N,N'-[6-(methoxycarbonyl)pyridin-2-yl]-methyl-1,2-diaminoethane), HP-D03A (Hydroxypropyltetraazacyclododecanetriacetic acid), and porphyrin. A compound represented by the following formula (A) is preferred.

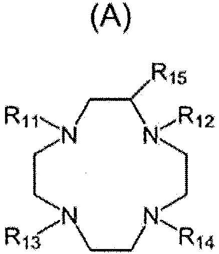

In the formula (A), R₁₁, R₁₂, R₁₃ and R₁₄ are each independently a group consisting of -(CH₂)ₚCOOH, -(CH₂)ₚC₅H₅N, -(CH₂)ₚPO₃H₂, - (CH₂)ₚCONH₂ or -(CHCOOH) (CH₂)ₚCOOH, R₁₅ is a hydrogen atom, and p is an integer of not less than 0 and not more than 3.

The compound represented by the formula (A) is preferably a compound containing a structure derived from 1,4,7,10-tetraazacyclododecane-1,4,7,10-tetraacetic acid (DOTA), or a derivative thereof. Specifically, the compound can contain, in its structure, a structure derived from one chelating agent selected from DOTA (1,4,7,10-Tetraazacyclododecane-1,4,7,10-tetraacetic acid), DOTMA ((1R,4R,7R,10R)-α,α',α",α‴-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTPA (1,4,7,10-tetraazacyclododecane-1,4,7,10-tetrapropionic acid), 1,4,7,10-tetrakis(pyridin-2-ylmethyl)-1,4,7,10-tetraazacyclododecane (L^{py}), DOTA-GA (α-(2-Carboxyethyl)-1,4,7,10-tetraazacyclododecane-1,4,7,10-tetraacetic acid), DOTP (((1,4,7,10-Tetraazacyclododecane-1,4,7,10-tetrayl)tetrakis(methylene))tetraphosphonic acid), DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), and D02P (Tetraazacyclododecane dimethanephosphonic acid). More preferably, compounds represented by the following formulas (A-1) to (A-6) can be mentioned. The chelating agent to be used in the radioconjugate of the present invention is further preferably DOTA-GA (compound represented by the formula (A-6)).

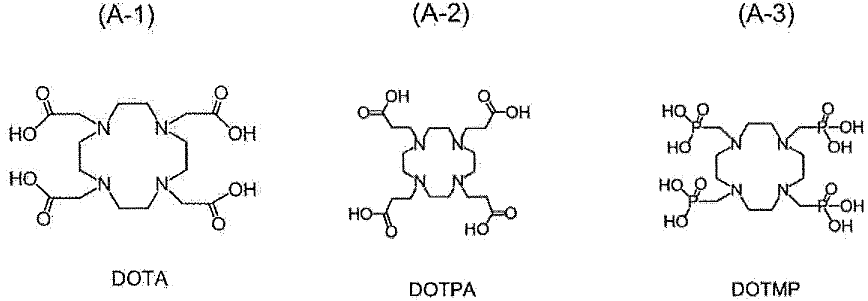

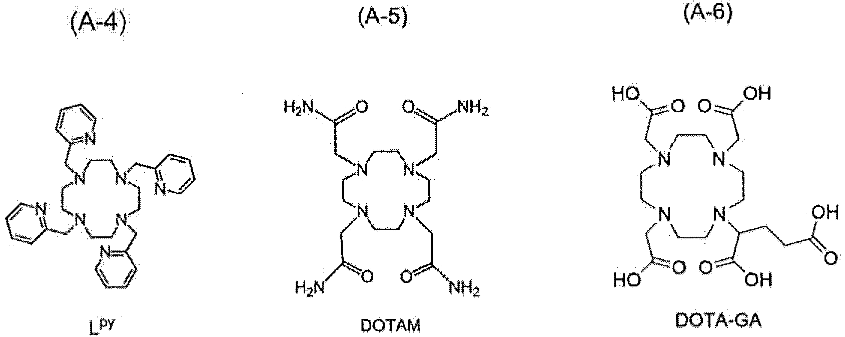

In the conjugate of the present invention, the chelating agent may be linked to an antibody via linker (L). Examples of the linker (L) include substituted or unsubstituted alkyl group, substituted or unsubstituted heteroalkyl group, polyethylene glycol (PEG) group, peptide, sugar chain, disulfide group, combination of these and the like.

In the conjugate of the present invention, the chelating agent and the linker (L) are preferably linked by a covalent bond. Therefore, in the conjugate of the present invention, some groups in the compound of the aforementioned chelating agent may be substituted by groups that form covalent bonds with the linker (L). For example, when the chelating agent used in the present invention is a compound represented by the formula (A), R₁₂ or R₁₅ may be substituted by a group that forms a covalent bond with the linker (L). Preferably, when R₁₂ is substituted by a group that forms a covalent bond with the linker (L), R₁₅ is a hydrogen atom, when R₁₂ is a group consisting of -(CH₂)ₚCOOH, -(CH₂)ₚC₅H₅N, -(CH₂)ₚPO₃H₂, -(CH₂)ₚCONH₂, or - (CHCOOH) (CH₂)ₚCOOH, R₁₅ is substituted by a group that forms a covalent bond with the linker (L).

The linking between the chelating agent and the linker (L) can be formed, for example, by the reaction of an N-hydroxysuccinimide ester (NHS) group of the following formula (A-7) or (A-8), or a 2,6-dioxotetrahydro-2H-pyranyl group of the following formula (A-9), with the primary amine of linker (L) .

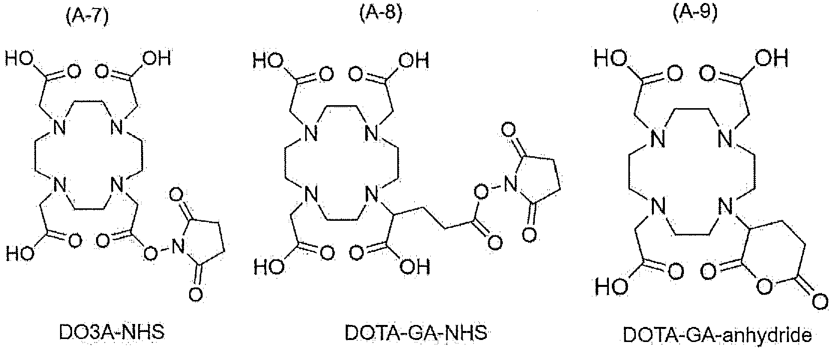

In the conjugate 1 of the present invention, at least one molecule of the chelating agent may be contained for one molecule of the antibody. From the aspects of maintaining the activity of the antibody itself (antigen recognition action, neutralizing action, complement activating action and/or opsonin action), a chelating agent is preferably introduced site-specifically into the Fc region (constant region) of the antibody. In the conjugate 1 of the present invention, it is more preferable to contain one molecule or two molecules of the chelating agent for each molecule of the antibody.

### (1-4) Antibody-modification peptide

Preferably, the deglycosylated antibody used in the present invention is site-specifically, preferably in the Fc region, modified with an antibody-modification peptide. In this case, the chelating agent that can be used in the present invention contains, via linker (L), a peptide consisting of not less than 13 and not more than 17 amino acid residues represented by the following formula (i) (hereinafter to be also referred to as "antibody-modification peptide"), and one formed by a crosslinking reaction of an antibody-modification peptide modified with a crosslinking agent and an antibody to be used. In the explanation of formula (i), the left side of the paper surface of the amino acid sequence indicates the N-terminal side, and the right side of the paper surface of the amino acid sequence indicates the C-terminal side. When the chelating agent is linked to the antibody via the antibody-modification peptide as a linker, the position where the chelating agent and the antibody-modification peptide are linked is not particularly limited and, for example, it may be directly or indirectly linked at the N-terminal or C-terminal, preferably N-terminal, of the antibody-modification peptide. Furthermore, the C-terminal of the antibody-modification peptide may be modified by amidation and the like in order to improve its stability and the like.

(Xa)-Xaa1-(Xb)-Xaa2-(Xc)-Xaa3-(Xd)··· (i)

In the formula (i), Xa, Xb, Xc and Xd are each continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy a+b+c+d≤14,
Xaa1 and Xaa3 are each independently
an amino acid residue derived from an amino acid having a thiol group in the side chain, or
one is an amino acid residue derived from an amino acid having a thiol group in the side chain and the other is an amino acid residue derived from an amino acid having a haloacetyl group in the side chain, and Xaa1 and Xaa3 are bonded, and
Xaa2 is a lysine residue, arginine residue, cysteine residue, aspartic acid residue, glutamic acid residue, 2-aminosuberic acid, or diamino propionic acid, and is modified with a crosslinking agent.

Examples of the amino acid residue that may be contained in X in the above-mentioned formula (i) include those derived from amino acids such as glycine, alanine, phenylalanine, proline, asparagine, aspartic acid, glutamic acid, arginine, histidine, serine, threonine, tyrosine, methionine and the like, and X may be an amino acid residue consisting of the same type of amino acid, or different types of amino acids.

In the formula (i), a, b, c and d are not particularly limited as long as they are numbers within the aforementioned range. From the aspect of the stability of binding between the peptide and antibody, a+b+c+d≤14 is to be satisfied, and a is preferably an integer of not less than 1 and not more than 3, b is preferably an integer of not less than 1 and not more than 3, c is preferably an integer of not less than 3 and not more than 5, and d is preferably an integer of not less than 1 and not more than 3.

Xaa1 and Xaa3 is an amino acid residue derived from an amino acid having a thiol group in the side chain, and Xaa1 and Xaa3 may be the same or different. Examples of the amino acid having a thiol group in the side chain include cysteine and homocysteine. Such amino acid residues are preferably bonded by a disulfide bond, or a sulfide group is preferably bonded thereto via a linker shown by the following formula (4). In the formula (4), the wavy line indicates the binding part with the sulfide group.

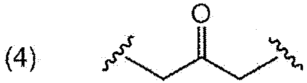

Instead of the aforementioned combination of Xaa1 and Xaa3, one of Xaa1 and Xaa3 may be an amino acid residue derived from an amino acid having a thiol group in the side chain, and the other may be an amino acid residue derived from an amino acid having a haloacetyl group in the side chain. These are bonded via a thioether bond. The terminal of the haloacetyl group is substituted with a halogen such as iodine or the like, and the halogen is eliminated by a reaction with the thiol group in the other side chain, whereby a thioether bond is formed.

Specific examples of the amino acid sequence of the antibody-modification peptide represented by the formula (i) include the peptides described in WO 2016/186206, WO 2017/217347 and WO 2018/230257, and these can also be used.

Among these, the amino acid sequence of the antibody-modification peptide preferably has any one of the following sequences (1) to (18), more preferably the following sequence (1), (2), (13) or (14). In the following amino acid sequences (1) to (18), (Xaa2) is a lysine residue, a cysteine residue, an aspartic acid residue, a glutamic acid residue, a 2-aminosuberic acid, or a diamino propionic acid, and (Xaa1) and (Xaa3) are each a homocysteine residue. Homocysteines may form a disulfide bond with each other. In the following amino acid sequences (1) to (18), the amino acids other than (Xaa1), (Xaa2) and (Xaa3) are indicated by one-letter abbreviations.

(1) DCAYH(Xaa2)GELVWCT (SEQ ID NO: 1),
(2) GPDCAYH(Xaa2)GELVWCTFH (SEQ ID NO: 2),
(3) RCAYH(Xaa2)GELVWCS (SEQ ID NO: 3),
(4) GPRCAYH(Xaa2)GELVWCSFH (SEQ ID NO: 4),
(5) SPDCAYH(Xaa2)GELVWCTFH (SEQ ID NO: 5),
(6) GDDCAYH(Xaa2)GELVWCTFH (SEQ ID NO: 6),
(7) GPSCAYH(Xaa2)GELVWCTFH (SEQ ID NO: 7),
(8) GPDCAYH(Xaa2)GELVWCSFH (SEQ ID NO: 8),
(9) GPDCAYH(Xaa2)GELVWCTHH (SEQ ID NO: 9),
(10) GPDCAYH(Xaa2)GELVWCTFY (SEQ ID NO: 10),
(11) SPDCAYH(Xaa2)GELVWCTFY (SEQ ID NO: 11),
(12) SDDCAYH(Xaa2)GELVWCTFY (SEQ ID NO: 12),
(13) RGNCAYH(Xaa2)GQLVWCTYH (SEQ ID NO: 13),
(14) G(Xaa1)DCAYH(Xaa2)GELVWCT(Xaa3)H (SEQ ID NO: 14)
(15) DCTYH(Xaa2)GNLVWCT (SEQ ID NO: 15),
(16) DCAYH(Xaa2)GNLVWCT (SEQ ID NO: 16),
(17) DCTYH(Xaa2)GELVWCT (SEQ ID NO: 17), and
(18) DCAWH(Xaa2)GELVWCT (SEQ ID NO: 18).

### (1-5) Linker (L)

Linker (L) is not particularly limited as long as it can link a chelating agent and a peptide in the radioconjugate of the present invention. Examples thereof include substituted or unsubstituted alkyl group, substituted or unsubstituted heteroalkyl group, polyethylene glycol (PEG) group, peptide, sugar chain, disulfide group, amide group, combination of these and the like.

In the present specification, linker (L) is a general term for linkers used for the connection of a deglycosylated antibody modified with a peptide and a chelating agent, and includes antibody-modification linker (L₁) and chelate linker (L₂). The antibody-modification linker (L₁), which is described in detail later, is to be introduced into the N-terminal side of the peptide described in (1-4), and the chelate linker (L₂), which is described in detail later, is to be introduced into the functional group of the chelating agent described in (1-3).

The linker (L) used in the present invention may contain a binding site formed by a click reaction, and preferably, the antibody-modification linker (L₁) and the chelate linker (L₂) are bound by a click reaction. As used herein, the binding site formed by the click reaction is preferably a triazole skeleton-containing structure represented by the following formula (10a) or (10b) or a pyridazine skeleton-containing structure represented by the following formula (10c) can be considered. Since the formula (10a) and the formula (10b) are isomers, they may be contained at any ratio.

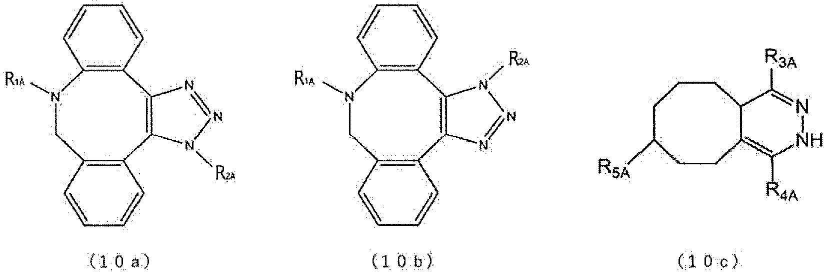

In the formula (10a) and the formula (10b), R_{1A} is a linking site with a chelating agent, R_{2A} is a linking site with an antibody-modification peptide. In the formula (10c), one of R_{3A} and R_{4A} is a hydrogen atom, a methyl group, a phenyl group or a pyridyl group, and the other is a linking site with a chelating agent, and R_{5A} is a linking site with an antibody-modification peptide. In the formula (10a), the formula (10b), and the formula (10c), the linking site with the antibody-modification peptide is linked with the peptide via the antibody-modification linker (L₁), and the linking site with the chelating agent is linked with the chelating agent via the chelate linker (L₂) .

In the radioconjugate of the present invention, the antibody is site-specifically modified with a peptide, and. the peptide and a chelating agent are linked via a linker (L). Thus, one molecule or two molecules of the chelating agent are conjugated to one molecule of the deglycosylated antibody.

### (1-6) Method for producing conjugate

The method for producing the conjugate of the present invention includes two steps which are a conjugation step of conjugating a chelating agent and an antibody, and a complex formation step of forming a complex of a radionuclide and a chelating agent. The conjugation step may be performed before the complex formation step or after the complex formation step.

In the conjugation step, various site-specific chemical modification methods for antibodies can be used. For example, a method of site-specifically modifying the Fc region of an antibody with a chelating agent or linker having the antibody-modification peptide shown in the aforementioned (1-4) can be mentioned.

In the complex formation step, the chelating agent is chelated with a metal radionuclide (complex formation). The metal radionuclide used here is preferably used in a manner permitting ionization, more preferably in the form of an ion, from the viewpoint of increasing the complex formation efficiency. In the complex forming step, the order of addition of the metal radionuclide to the chelating agent does not matter as long as a complex can be formed with the metal radionuclide. For example, a solution in which radioactive metal ions are dissolved in a solvent mainly composed of water can be used as a radionuclide.

After complex formation, the obtained complex may be purified using a filtration filter, a membrane filter, a column filled with various fillers, chromatography or the like.

In the method for producing the conjugate of the present invention, a conjugation step is preferably performed after the complex formation step.

In a more preferred embodiment, in complex formation step (A), a complex is formed between a radionuclide and a chelating agent having a first atomic group capable of click reaction as a substituent for enabling conjugate formation with the antibody. Then, in conjugation step (B), using an antibody-modification peptide shown by the aforementioned formula (i) and an antibody-modification linker (L₁) having a second atomic group capable of click reaction, a click reaction is performed between the peptide-modified antibody in which Fc region is site-specifically modified and the chelating agent with a formed complex which is obtained in step (A) to obtain the conjugate of the present invention.

The steps (A) and (B) are described in detail below.

As the combination of the first atomic group and the second atomic group capable of click reaction, an appropriate combination is selected according to the type of the click reaction. For example, a combination of alkyne and azide, a combination of 1,2,4,5-tetrazine and alkene, and the like can be mentioned. In these atomic groups, the first atomic group has one of the above-mentioned atomic group combination, and the second atomic group has one atomic group which is different from the first atomic group of the above-mentioned atomic group combination. To achieve both the stability of the chelating agent and the antibody and the improvement of the binding efficiency thereof, the chelate linker (L₂) is preferably alkyne and the antibody-modification linker (L₁) is preferably azide, or the chelate linker (L₂) is preferably 1,2,4,5-tetrazine and the antibody-modification linker (L₁) is preferably alkene. Specific examples of the click reaction by such combinations of atomic groups include a Husgen cyclization addition reaction, an inverse electron-requested Diels-Alder reaction, and the like.

Specific examples of the combination of the atomic groups capable of click reaction include, as shown in the following formulas, a combination of an atomic group containing dibenzylcyclooctyne (DBCO) as alkyne of the first atomic group (the formula (1a)) and an atomic group containing an azide group as azide of the second atomic group (the formula (2a)), or else a combination of an atomic group containing 1,2,4,5-tetrazine as the first atomic group (the formula (1b)) and an atomic group containing trans-cyclooctene (TCO) as alkene of the second atomic group (the formula (2b)). A combination of the formula (1a) and the formula (2a) is preferred.

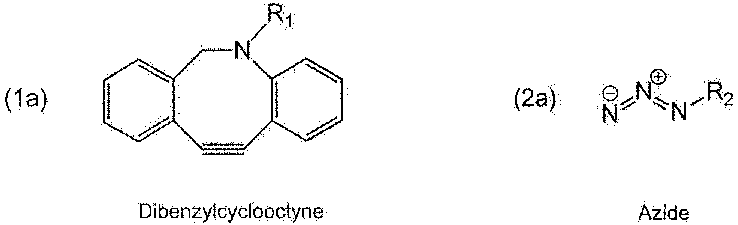

In the formula (1a), R₁ is a linking site with a chelating agent, and in the formula (2a), R₂ is a linking site with an antibody-modification peptide in the antibody.

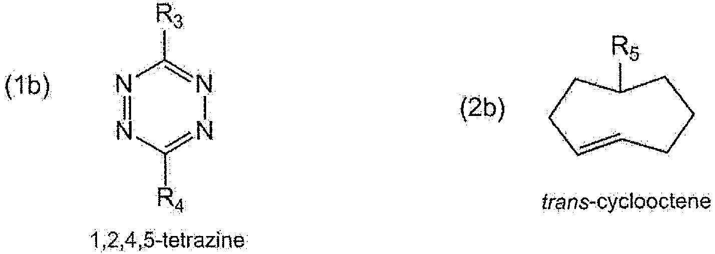

In the formula (1b), one of R₃ and R₄ is a linking site with one of a chelating agent or an antibody-modification peptide in the antibody, and the other is a hydrogen atom, a methyl group, a phenyl group or a pyridyl group, and in the formula (2b), R₅ is a linking site with either a chelating agent or an antibody-modification peptide in the antibody, depending on R₃ or R₄.

When an atomic group containing DBCO represented by the above-mentioned formula (1a) as alkyne of the first atomic group is used, various commercially available DBCO reagents can be mentioned. Specifically, for example, DBCO-C6-Acid, Dibenzylcyclooctyne-Amine, Dibenzylcyclooctyne Maleimide, DBCO-PEG acid, DBCO-PEG-NHS ester, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, DBCO-mPEG and the like can be selected, and Dibenzylcyclooctyne Maleimide is preferably used.

In step (A), more preferably, a chelating agent having a structure represented by the following formula (ii) is used.

A-B-C... (ii)

In the formula (ii), A is a chelate site represented by the following formula (iia).

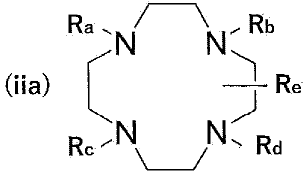

In the formula (iia), Ra, Rb and Rc are each independently a group consisting of -(CH₂)ₚCOOH, -(CH₂)ₚC₅H₅N, - (CH₂)ₚPO₃H₂, -(CH₂)ₚCONH₂ or - (CHCOOH) (CH₂)ₚCOOH, p is an integer of not less than 0 and not more than 3, one of Rd and Re is a binding site (*) with B, and other is a hydrogen atom or a group consisting of -(CH₂)ₚCOOH, -(CH₂)ₚC₅H₅N, -(CH₂)ₚPO₃H₂, - (CH₂)ₚCONH₂ or, -(CHCOOH) (CH₂)ₚCOOH, and p is an integer of not less than 0 and not more than 3.

In the formula (ii), B is represented by the following formula (iib).

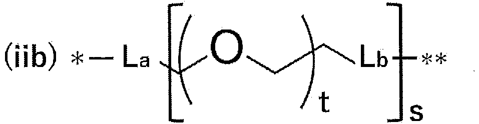

In the formula (iib), La and Lb are each independently a bond linker containing at least an amide bond or a thiourea bond and not less than 1 and not more than 50 carbon atoms, t is an integer of not less than 0 and not more than 30, s is 0 or 1, * is a binding site with A, and ** is a binding site with C.

In the formula (ii), C is either an alkyne derivative represented by the following formula (iic) or a tetrazine derivative represented by the formula (iid).

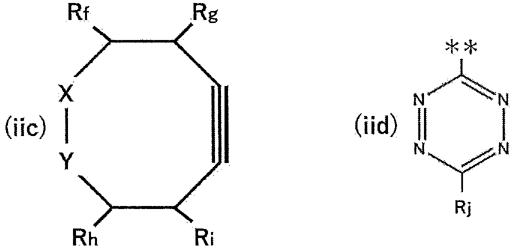

In the formula (iic), X is CHRk-** or N-**, Y is CHRk or C=O, Rk is independently a hydrogen atom or an alkyl group having not less than 1 and not more than 5 carbon atoms, when X is CHRk-** and Y is CHRk, then Rk moieties may be joined to form a cycloalkyl group, Rf, Rg, Rh and Ri are each independently a hydrogen atom, a halogen atom, or an alkyl group having not less than 1 and not more than 5 carbon atoms, Rf and Rg may be joined, or Rh and Ri may be joined to form a hydrocarbon ring, ** is a binding site with B, in the formula (iid), ** is a binding site with B, and Rj is a hydrogen atom, a methyl group, a phenyl group or a pyridyl group.

As the chelating agent used in step (A), a DOTA derivative of the above-mentioned formula (iia) wherein Ra to Rd are -(CH₂)ₚCOOH, p is 1, Re is a binding site (*) with B; or DO3A derivative or DOTAGA derivative wherein Ra to Rc are - (CH₂)ₚCOOH, p is 1, Rd is a binding site (*) with B, and Re is a hydrogen atom is more preferred.

In the formula (ii), when A is the above-mentioned DOTA derivative, a DOTA-PEGt-DBCO derivative wherein, in B, La is a bond linker containing a thiourea bond and having not less than 1 and not more than 50 carbon atoms, s is 0 or 1, when s is 1, t is an integer of not less than 0 and not more than 30, Lb is a bond linker containing an amide bond or a thiourea bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B; or a DOTA-PEGt-Tz derivative wherein, in B, La is a bond linker containing a thiourea bond and having not less than 1 and not more than 50 carbon atoms, s is 0 or 1, when s is 1, t is an integer of not less than 0 and not more than 30, Lb is a bond linker containing an amide bond or a thiourea bond and having not less than 1 and not more than 50 carbon atoms, and C is a tetrazine derivative represented by the formula (iid), is further more preferred.

In the formula (ii), when A is the above-mentioned DO3A derivative, a DO3A-PEGt-DBCO derivative wherein, in B, La is a bond linker containing an amide bond or a thiourea bond and having not less than 1 and not more than 50 carbon atoms, s is 0 or 1, when s is 1, t is an integer of not less than 0 and not more than 30, Lb is a bond linker containing an amide bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is further more preferred.

In the formula (ii), when A is the above-mentioned DOTAGA derivative, a DOTAGA-PEGt-DBCO derivative wherein, in B, La is a bond linker containing an amide bond or a thiourea bond and having not less than 1 and not more than 50 carbon atoms, s is 0 or 1, when s is 1, t is an integer of not less than 0 and not more than 30, Lb is a bond linker containing an amide bond or a thiourea bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is further more preferred.

In the molar ratio of the chelating agent and radionuclide as chelate site/radionuclide, the lower limit is preferably not less than 10/1, more preferably not less than 100/1, further preferably not less than 500/1, and the upper limit is preferably not more than 15000/1, more preferably not more than 10000/1, further preferably not more than 7000/1. For example, the range of not less than 100/1 and not more than 7000/1 is preferred, and not less than 500/1 and not more than 7000/1 is more preferred.

The complex formation reaction is preferably performed in a solvent. As the solvent, for example, water, saline, or buffer such as sodium acetate buffer, ammonium acetate buffer, phosphate buffer, phosphate buffered saline, tris hydroxymethylaminomethane buffer (Tris buffer), 4-(2-hydroxyethyl)-1-piperazineethanesulfonic acid buffer (HEPES buffer), tetramethylammonium acetate buffer or the like can be used.

While the amount of the solvent is not particularly limited, from the aspect of practicality in the production step, at the start of step (A), the lower limit is not less than 0.01 mL, preferably not less than 0.1 mL, more preferably not less than 1.0 mL, further preferably not less than 10 mL, further more preferably not less than 100 mL, and upper limit is preferably not more than 1000 mL, more preferably not more than 100 mL, further preferably not more than 10 mL, further more preferably not more than 1.0 mL. For example, it is within the range of not less than 0.01 mL and not more than 100 mL.

As the concentration of the chelating agent in the reaction mixture of the complex formation reaction, from the aspect of the yield of the desired chelating agent, at the start of step (A), the lower limit is each independently preferably not less than 0.001 µmol/L, more preferably not less than 0.01 µmol/L, further preferably not less than 0.1 µmol/L, more preferably not less than 1 µmol/L, and the upper limit is preferably not more than 1000 µmol/L, more preferably not more than 100 µmol/L, further preferably not more than 10 µmol/L. For example, it is within the range of not less than 1 µmol/L and not more than 100 µmol/L.

The temperature of the complex formation reaction may be, for example, room temperature (25°C) or under heating conditions. To simultaneously achieve suppression of decomposition of the chelating agent and improvement of complex formation efficiency, the lower limit is preferably not less than 20°C, more preferably not less than 30°C, further preferably not less than 35°C, further more preferably not less than 37°C, particularly preferably not less than 45°C. The upper limit is preferably not more than 150°C, more preferably not more than 120°C, further preferably not more than 100°C, further more preferably not more than 90°C. For example, a range of not less than 30°C and not more than 100°C is preferred, and a range of not less than 35°C and not more than 90°C is more preferred.

On the condition that the aforementioned reaction temperature applies, the lower limit of the reaction time is preferably not less than 5 min, more preferably not less than 10 min, further preferably not less than 20 min, further more preferably not less than 30 min, particularly preferably not less than 45 min. The upper limit is preferably not more than 180 min, more preferably not more than 150 min, further preferably not more than 120 min, further more preferably not more than 90 min, particularly preferably not more than 60 min and, for example, the range of not less than 10 min and not more than 150 min is preferred, and the range of not less than 10 min and not more than 60 min is more preferred.

The antibody to be used in step (B) is a peptide-modified antibody in which Fc region (constant region) of humanized antibody as described in detail in the above-mentioned "(1-2) Antibody" is site-specifically modified using the antibody-modification peptide shown in the aforementioned formula (i), and an antibody-modification linker (L₂) having the second atomic group capable of click reaction.

The antibody-modification peptide can be produced using a combination of amino acids regardless of natural amino acids and unnatural amino acids, by subjecting to peptide synthesis methods such as liquid phase synthesis process, solid phase synthesis process, automatic peptide synthesis method, gene recombinant method, phage display method, and the like. In the synthesis of the peptide, where necessary, the functional groups of the amino acids to be used may be protected. These methods can be performed according to the method described in, for example, WO 2017/217347 and WO 2018/230257.

The antibody-modification linker (L₂) may be one in which an antibody-modification peptide and a linker represented by the following formula (S1) are bonded.

*-((L₁)ₘ-Z)ₖ-L₂-AG₂... (S1)

wherein * is a binding site with the N-terminal or C-terminal of a peptide,
L₁ is a linker site of polyethylene glycol (PEG),
m is an integer of not less than 1 and not more than 50,
Z is a second linker site that binds (L₁)ₘ and L₂,
k is 0 or 1,
L₂ is a second PEG linker site, and
AG₂ is a second atomic group.

In the aforementioned formula (S1), the structure of Z is not particularly limited as long as it is a linker structure that binds (L₁)ₘ and L₂ to each other, and includes, for example, an amino acid sequence consisting of not less than 1 and not more than 5 amino acid residues. In this case, the amino acid sequence contained in Z preferably contains a cysteine residue, and is more preferably bonded to L₂ via a thioether group formed by the bond between the thiol group of the cysteine residue and a maleimide group.

In the present invention, the PEG linker site constituting L₂ preferably has the structure shown by the following formula (P2). In the formula (P2), n is an integer of preferably not less than 1 and not more than 50, more preferably not less than 1 and not more than 20, further preferably not less than 2 and not more than 10, further more preferably not less than 2 and not more than 6.

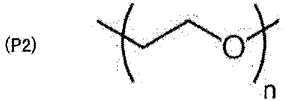

One end of the structure of the PEG linker site may be modified by a structure derived from a commercially available PEGylation reagent or a structure derived from a reagent generally used for PEGylation. Although not particularly limited, examples thereof include structures derived from diglycolic acid or a derivative thereof, and maleimide or a derivative thereof.

As a method for introducing the aforementioned second atomic group into the antibody-modification linker (L₂), an introduction method including obtaining an antibody-modification peptide having a desired amino acid sequence by the aforementioned method, dissolving the peptide in a solution containing a solubilizing agent and a reducing agent and where necessary, an acid, adding an organic solvent solution of an atomic group containing an azide group or trans-cyclooctene (TCO) as the second atomic group to the solution, and stirring the mixture at room temperature can be mentioned.

When an atomic group containing an azide group is introduced as the second atomic group, the azide group is directly introduced into the N-terminal or C-terminal of a peptide by using a commercially available azide group-introducing reagent according to a conventional method, or an atomic group containing an azide group can be introduced via the aforementioned linker structure. Examples of the azide group-introducing reagent to be used include silyl azide, azide phosphate, alkyl ammonium azide, inorganic azide, sulfonyl azide, PEG azide, and the like.

When an atomic group containing TCO is introduced as the second atomic group, TCO is directly introduced into the N-terminal or C-terminal of a peptide by using a commercially available click chemistry reagent containing TCO according to a conventional method, or an atomic group containing TCO can be introduced via the aforementioned linker structure.

The method for binding an antibody-modification peptide to a deglycosylated antibody to obtain a peptide-modified antibody can be performed, for example, by dispersing the aforementioned antibody-modification peptide, a deglycosylated antibody, a crosslinking agent, and a catalyst as necessary in an appropriate buffer, according to the description of WO 2017/217347. As the crosslinking agent, those mentioned above can be used. In addition, when binding the antibody-modification peptide and the deglycosylated antibody, where necessary, buffer replacement including treating the solution containing the deglycosylated antibody with an ultrafiltration filter, etc., and dispersing same in a buffer may be performed once or twice before binding with the antibody-modification peptide.

The antibody-modification peptide of the present invention binds to an antibody, for example, Fc domain of IgG. In this case, the antibody-modification peptide of the present invention can also be said an IgG binding peptide. At the above-mentioned Xaa1, the IgG binding peptide of the present disclosure is close to a specific region of IgG Fc, that is, a lysine residue in the Fc region corresponding to the Lys248 residue or Lys246 residue, preferably Lys248 residue, according to Eu numbering in human IgG Fc. Therefore, by modifying Xaa1 of the IgG binding peptide of the present disclosure with a crosslinking agent and causing a crosslinking reaction with IgG, Xaa1 of the IgG binding peptide can site-specifically form a crosslinking structure with the lysine residue of the IgG Fc of the present disclosure that corresponds to the above-mentioned lysine residue.

In the present disclosure, the "crosslinking agent" is a chemical substance to link the IgG-binding peptide of the present disclosure and IgG Fc by a covalent bond. The crosslinking agent of the present disclosure can be appropriately selected by those of ordinary skill in the art, and can be a compound having at least two sites bindable to desired amino acid residue (e.g., lysine residue, cysteine residue, aspartic acid residue, glutamic acid residue, 2-aminosuberic acid, diaminopropionic acid, arginine residue, etc.). Examples thereof include, but are not limited to, a crosslinking agent preferably containing two or more succinimidyl groups such as DSG (disuccinimidyl glutarate), DSS (disuccinimidyl suberate), and the like, a crosslinking agent preferably containing two or more imide acid moieties such as DMA (dimethyl adipimidate·2HCl, dimethyl adipimidate dihydrochloride), DMP (dimethyl pimelimidate·2HCl, dimethyl pimelimidate dihydrochloride), DMS (dimethyl suberimidate·2HCl, dimethyl suberimidate dihydrochloride), and the like, and a crosslinking agent having an SS bond such as DTBP (dimethyl 3,3'-dithiobispropionimidate·2HCl, dimethyl 3,3'-dithiobispropionimidate dihydrochloride) and DSP (dithiobis(succinimidyl propionate)), and the like.

For example, a crosslinking agent containing a succinimidyl group such as DSS or DSG reacts with primary amine present in the side chain of lysine residue and the N-terminal of polypeptide. Therefore, only the side chain of the lysine residue can be specifically modified with DSS or DSG by blocking the N-terminal of the IgG-binding peptide and reacting same with DSS or DSG. Those of ordinary skill in the art can appropriately select such combination of an amino acid residue and a crosslinking agent.

A crosslinking reaction between IgG-binding peptide and the antibody of the present disclosure may site-specifically occur particularly between the above-mentioned amino acid residue of Xaa1 of the IgG-binding peptide and EU numbering Lys248 or Lys246, preferably Lys248, in human IgG Fc.

The conditions of the mixing step are not particularly limited as long as a crosslinking reaction occurs between the IgG-binding peptide of the present disclosure and the antibody of the present disclosure. For example, the reaction can be performed by mixing the IgG-binding peptide of the present disclosure and the antibody of the present disclosure in an appropriate buffer at room temperature (e.g., about 15°C to 30°C). The mixing step may be performed by adding as necessary an appropriate amount of a catalyst that promotes the crosslinking reaction.

In one embodiment, a solvent containing at least water is added to dissolve the antibody of the present disclosure. The solvent other than water includes, for example, dimethyl sulfoxide, acetonitrile, saline, and buffers such as sodium acetate buffer, ammonium acetate buffer, phosphate buffer, phosphate buffered saline, Tris buffer, HEPES buffer, tetramethylammonium acetate buffer, and the like. When a buffer is used, the pH at 25°C is preferably set to 4.0 or more and 10.0 or less, more preferably 5.5 or more and 8.5 or less, from the aspect of the stability of the antibody. At the start of the crosslinking reaction, the concentration of the antibody is preferably set to 1.0 µmol/L or more as the lower limit and 1000 µmol/L or less, preferably 500 µmol/L or less, as the upper limit.

Then, an IgG-binding peptide modified with a crosslinking agent and, where necessary, a catalyst are added and the mixture is dispersed at 10°C or higher and 30°C or lower.

The mixing ratio of the IgG-binding peptide of the present disclosure and the antibody of the present disclosure in the mixing step is not particularly limited. The molar ratio of the IgG-binding peptide of the present disclosure and the antibody of the present disclosure can be set to, for example, 1:1 to 20:1, preferably 2:1 to 20:1 or 5:1 to 10:1.

In a preferred embodiment, IgG-binding peptide can be mixed with the antibody of the present disclosure at 0.5 to 2.2, preferably 0.8 to 1.8, (molar ratio) in the above-mentioned mixing step. In this way, a monovalent modified antibody (that is, conjugate containing one IgG-binding peptide for antibody) can be obtained efficiently.

The mixing time (reaction time) in the mixing step is not particularly limited as long as a crosslinking reaction occurs between the IgG-binding peptide of the present disclosure and the antibody of the present disclosure. It is, for example, 1 min to 5 hr, preferably 10 min to 2 hr.

The peptide-modified antibody obtained through the above steps is a mixture containing an antibody in which one molecule of antibody-modification peptide is bound to one molecule of deglycosylated antibody (hereinafter referred to as "monovalent antibody") and an antibody in which two molecules of antibody-modification peptide are bound to one molecule of deglycosylated antibody (hereinafter referred to as "divalent antibody") at any ratio. This may be used as it is for the subsequent steps, or an unmodified antibody, a monovalent antibody, and a divalent antibody are separated and purified by a method such as filtration filter, membrane filter, column filled with various fillers, various chromatographies and the like, and only the antibody having any valence may be subjected to the subsequent steps.

When the unmodified antibody cannot be separated from the antibody having other valence as a result of purification, a mixture containing these may be subjected to the subsequent steps.

When an unmodified antibody, a monovalent antibody, and a divalent antibody are separated and purified, any of the above-mentioned purification methods may be used for separation and purification. It is possible to use a column packed with various fillers. For example, a column packed with a filler in which a protein such as protein A, protein G, or the aforementioned antibody-modification peptide is bound to a carrier can be used. The shape of the carrier of the filler packed in such a column includes gel (e.g., column gel), particle, bead, nanoparticle, microparticle, macrobead, and the like. The materials of the carrier include magnetic substance, latex, agarose, glass, cellulose, sepharose, nitrocellulose, polystyrene, and other polymeric materials. Specific example is an IgG-BP column in which the aforementioned antibody-modification peptide is bound to a column gel (see WO 2021/080008).

Using the IgG-BP column and utilizing the difference in the interaction with respective antibody-modification peptides, the first antibody composition containing relatively large amounts of the unmodified antibody and the monovalent antibody and the second antibody composition containing a relatively large amount of the divalent antibody can be respectively separated and purified. In one preferred embodiment, the molar ratio of unmodified antibody to monovalent modified antibody in the first antibody composition is 4-47:53-96, preferably 4-30:70-96, more preferably 4-20:80-96, further preferably 4-10:90-96.

The first antibody composition or the second antibody composition separated and purified may be used as it is for the click reaction in the subsequent step (B), or may be used for the click reaction in step (B) after adjusting the protein concentration of the peptide-modified antibody contained.

The click reaction in step (B) is performed between the first atomic group capable of click reaction which is contained in the chelating agent, and the second atomic group capable of click reaction which is contained in the peptide-modified antibody. By such click reaction, a binding group (substituent capable of conjugating with antibody) that links a chelating agent and an antibody is formed.

When the peptide-modified antibody and the complex obtained in step (A) are capable of click reaction, the order of addition of these does not matter. For example, one of the complex and the peptide-modified antibody is added to a reaction container containing a solvent, and then the other is added to perform the reaction, or one of the chelating agent and the antibody is dispersed in a solvent and the other is added to the dispersion to perform the reaction. Alternatively, these may be simultaneously added to a reaction container containing a solvent to perform the reaction.

As the solvent to be used for the click reaction in step (B), a solvent containing water can be used. For example, water, saline, buffers such as sodium acetate buffer, ammonium acetate buffer, phosphate buffer, phosphate buffered saline, Tris buffer, HEPES buffer, tetramethylammonium acetate buffer and the like, and the like can be used. When a buffer is used, to simultaneously achieve the stability of the complex and the antibody, and the bond efficiency of these, the pH at 25°C is preferably set to not less than 4.0 and not more than 10.0, further preferably not less than 5.5 and not more than 8.5.

While the amount of the reaction mixture is not particularly limited, from the aspect of practicality in the production step, at the start of step (B), the lower limit is preferably not less than 0.001 mL, more preferably not less than 0.01 mL, further preferably not less than 0.1 mL, further more preferably not less than 1 mL, and the upper limit is preferably not more than 1000 mL, more preferably not more than 100 mL, further preferably not more than 10 mL, further more preferably not more than 1 mL. For example, the range of not less than 0.001 mL and not more than 1000 mL is preferable, and the range of not less than 0.1 mL and not more than 10 mL is more preferable.

As the concentrations of the chelating agent and the antibody in the reaction mixture, each independently, at the start of step (B), the lower limit is preferably not less than 0.001 µmol/L, more preferably not less than 0.01 µmol/L, further preferably not less than 0.1 µmol/L, further more preferably not less than 1.0 µmol/L, and the upper limit is preferably not more than 1000 µmol/L, more preferably not more than 100 µmol/L. For example, the range of not less than 0.1 µmol/L and not more than 1000 µmol/L is preferable, and the range of not less than 1 µmol/L and not more than 100 µmol/L is more preferable, from the aspect of the yield of the desired conjugate.

To prevent unintended denaturation of the antibody and increase the reaction efficiency, the upper limit of the reaction temperature of the click reaction in step (B) is preferably not more than 50°C, more preferably not more than 40°C. The lower limit of the reaction temperature is not particularly limited as long as the reaction proceeds, and is preferably not less than 15°C. The reaction time of the click reaction is, on the condition that it is the aforementioned reaction temperature, preferably not less than 5 min, more preferably not less than 10 min, preferably not more than 24 hr, more preferably not more than 20 hr. For example, the range of not less than 5 min and not more than 24 hr is preferable, and the range of not less than 10 min and not more than 20 hr is more preferable.

The obtained conjugate may be used as it is or purified using a filtration filter, a membrane filter, a column filled with various fillers, chromatography or the like.

In the conjugate produced by steps (A) and (B), the lysine residue in the Fc region of deglycosylated antibody is specifically modified with a chelating agent. This conjugate comprises one or two molecules of the aforementioned chelating agent per one molecule of the antibody. The chelating agent site-specifically modifies the Fc region of the antibody of the present invention via a linker (L). The linker (L) is constituted of a chelate linker (L₂) that connects to a chelating agent, a first atomic group that connects to the linker (L₂), a second atomic group that can perform click reaction with the first atomic group, and an antibody-modification linker (L₁) that connects to the second atomic group (including an antibody-modification peptide represented by the above-mentioned formula (i)). Therefore, the linker (L) has a chemical structure derived from the first atomic group and the second atomic group. As such chemical structure, a triazole skeleton-containing structure represented by the aforementioned formula (10a) or (10b) or a pyridazine skeleton-containing structure represented by the aforementioned formula (10c) can be considered. Since the formula (10a) and the formula (10b) are isomers, they may be contained at any ratio.

### (1-7) Radiopharmaceutical

A radiopharmaceutical refers to a composition that contains the conjugate of the present invention and is in a form suitable for in vivo administration to a subject. The radiopharmaceutical may be, for example, a conjugate produced by the method shown in the aforementioned (1-6) as it is, or can be produced by purifying same and dissolving same in a solvent mainly containing water and approximately isotonic with the living body. In this case, the radiopharmaceutical is preferably in the form of an aqueous solution, and may contain other pharmaceutically acceptable components as necessary. An effective amount of the radiopharmaceutical is orally or parenterally, for example, intravenously, subcutaneously, intraperitoneally, intramuscularly, or the like, administered to a living body, and is used for treatment of cancer, diagnosis of cancer, detection of a lesion, or the like.

As used herein, the subject of administration is a human, or an animal such as mouse, rat, monkey, guinea pig, chimpanzee, sheep, goat, dog, cat, swine, bovine, horse or the like, but is not particularly limited. Preferred is a human.

The target disease is determined according to the properties of the antibody used, and in the case of anti-HER2 antibody, diseases in which HER2 is overexpressed can be mentioned. Specifically, examples include breast cancer in which overexpression of HER2 has been confirmed, and advanced/recurrent gastric cancer in which overexpression of HER2 has been confirmed and which cannot be cured or resected. In the case of anti-EGFR antibody, diseases in which EGFR is overexpressed can be mentioned. Specific examples include unresectable advanced/recurrent colorectal cancer and head and neck cancer. In the case of anti-CD20 antibody, diseases in which CD20 is overexpressed can be mentioned. Specifically, suppression of CD20-positive B-cell non-hodykin lymphoma, CD20-positive B-cell lymphoproliferative disease under immunosuppressive conditions, Wegena's granulomatosis, microscopic polyangiitis, intractable nephrotic syndrome, antibody-related rejection in ABO blood type incompatible transplants during kidney and liver transplantations, and the like can be mentioned.

As used herein, the "effective amount" is an amount that can afford useful diagnostic or therapeutic effects in a subject of administration. The effective amount to be administered to a subject varies depending on the type of subject, body weight of the subject, dosage form (tablet, injection, etc.) and route (oral administration, parenteral administration, etc.) of administration, severity of disease (e.g., cancer), and the like. Physicians and veterinarians can consider these factors and determine the appropriate effective amount.

The radiopharmaceutical of the present invention can be used for radionuclide therapy of cancer by selecting metal radionuclides that have a therapeutic effect, specifically nuclide that emits α rays or nuclide that emits β rays (preferably Ac-225, Y-90, Lu-177, more preferably Ac-225). In this radionuclide therapy, the radiopharmaceutical of the present invention is administered by intravenous injection or orally to cause accumulation of the radioconjugate of the present invention in a lesion site such as a cancer primary lesion or metastatic lesion, and cancer cells at the lesion site are destroyed by the radiation emitted from the metal radionuclide. The amount to be administered and dose of the radiopharmaceutical of the present invention is appropriately determined by the efficacy of the active ingredient, the mode and route of administration, the stage of cancer progression, the body type, body weight and age of the patient, and the type and amount of the therapeutic drug used in combination for other diseases.

In addition, by selecting a radionuclide that emits positron or a radionuclide that emits γ rays (preferably Zr-89) as the metal radionuclide, it can be used for cancer diagnosis or lesion detection. A radiopharmaceutical using radionuclide that emits positron can be preferably used for PET (Positron Emission Tomography) examination, and a radiopharmaceutical using radionuclide that emits γ-rays can be preferably used for SPECT (Single Photon Emission Computed Tomography) examination. This may also be used in combination with cancer diagnosis or lesion detection in the aforementioned radionuclide therapy of cancer. The diagnostic radiopharmaceutical for cancer of the present invention may be used for diagnosis before performing radionuclide therapy for cancer, or may be used for diagnosis after performing radionuclide therapy for cancer. Using the radiopharmaceutical for diagnosis before conducting a radionuclide therapy for cancer, selection of treatment can be performed based on whether or not to perform radionuclide therapy for cancer using the radiopharmaceutical of the present invention provided with a radionuclide that emits α-rays. Using the radiopharmaceutical for diagnosis after conducting a radionuclide therapy for cancer, moreover, whether or not radionuclide therapy for cancer using the radiopharmaceutical of the present invention is effective can be judged, and a treatment plan including an increase or a decrease of dose and the like can be optimized.

### (2) Conjugate 2

In another embodiment, the present invention provides a conjugate of a chelating agent chelated with a radionuclide and a deglycosylated antibody, wherein the above-mentioned radionuclide is an α-ray nuclide.

In the conjugate of the present invention, the chelating agent may randomly modify the antibody, and in this case, it is preferable to contain not less than 1 molecule and not more than 8 molecules of the antibody.

When antibody is modified randomly, various methods for chemically modifying antibody are used in the conjugation step of a chelating agent or linker and the antibody. Specifically, methods (a) to (e) can be mentioned.
(a) amine coupling method (method for modifying the amino group of a lysine residue of an antibody by using a chelating agent or chelate having a carboxyl group activated with an N-hydroxysuccimidyl (NHS) group)
(b) method for modifying a sulfhydryl (SH) group generated by partially reducing a disulfide bond (SS bond) between polypeptide chains at the hinge site of an antibody with a chelating agent or linker having a maleimide group reactive with the SH group
(c) method for modifying cysteine newly introduced into an antibody by an amino acid mutation by genetic engineering with a chelating agent or linker having a maleimide group
(d) method for modifying an azide group of azidized lysine newly introduced into an antibody by an amino acid mutation by genetic engineering with a chelating agent or linker having alkyne (e.g., Dibenzylcyclooctyne: DBCO) by using a click reaction
(e) method for modifying glutamine introduced into a specific position of an antibody with a chelating agent or linker having a side chain of lysine by using transglutaminase

The same definition as in the above-mentioned "(1) Conjugate 1" applies, except that the radionuclide in the chelating agent is an α-ray nuclide, and the chelating agent may modify the antibody randomly.

The following embodiments are also encompassed in the technical idea of the present invention.
(1) A conjugate of a chelating agent chelated with a radionuclide and a deglycosylated antibody, wherein the chelating agent site-specifically modifies Fc region of the aforementioned antibody via a linker.
(2) The conjugate of (1), wherein the linker comprises an antibody-modification peptide composed of not less than 13 and not more than 17 amino acid residues represented by the following formula (i):

   (Xa)-Xaa1-(Xb)-Xaa2-(Xc)-Xaa3-(Xd)... (i)

   wherein Xa, Xb, Xc and Xd are continuous X in the number of a, continuous X in the number of b, continuous X in the number of c, and continuous X in the number of d, respectively,
   X is an amino acid residue having neither a thiol group nor a haloacetyl group in the side chain,
   a, b, c and d are each independently an integer of not less than one and not more than 5, and satisfy a+b+c+d≤14,
   Xaa1 and Xaa3 are each independently an amino acid residue derived from an amino acid having a thiol group in the side chain, and they are bonded via a disulfide bond or their sulfide groups are bonded via a linker, or
   one is an amino acid residue derived from an amino acid having a thiol group in the side chain and the other is an amino acid residue derived from an amino acid having a haloacetyl group in the side chain, and they are bonded via a thioether bond, and
   Xaa2 is a lysine residue, an arginine residue, a cysteine residue, an aspartic acid residue, a glutamic acid residue, 2-aminosuberic acid, or diamino propionic acid.)
(3) The conjugate of the above-mentioned (2), wherein the antibody-modification peptide is the aforementioned formula (i) wherein Xaa2 is a lysine residue.
(4) The conjugate of (2) or (3), wherein the antibody-modification peptide comprises an antibody-modification peptide consisting of the amino acid sequence shown in SEQ ID NO: 2 (wherein Xaa2 is a lysine residue).
(5) The conjugate of any one of (1) to (4), wherein the chelating agent has a structure derived from a compound represented by the following formula (A) or a salt thereof: 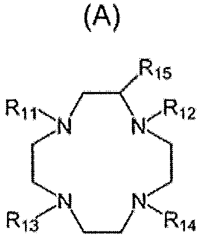 in the formula (A), R₁₁, R₁₃, and R₁₄ are each independently a group consisting of -(CH₂)ₚCOOH, -(CH₂)ₚC₅H₅N, -(CH₂)ₚPO₃H₂, - (CH₂)ₚCONH₂, or - (CHCOOH) (CH₂)ₚCOOH, one of R₁₂ and R₁₅ is a hydrogen atom, a carboxyl group, or a carboxyalkyl group having 2 or 3 carbon atoms, the other is a substituent for conjugating with the aforementioned antibody, p is an integer of not less than 0 and not more than 3, when R₁₂ is a substituent for conjugating with the aforementioned antibody, then R₁₅ is a hydrogen atom, and when R₁₂ is not a substituent for conjugating with the aforementioned antibody, then R₁₅ is a substituent for conjugating with the aforementioned antibody.
(6) The conjugate of any of (1) to (5), wherein the linker has a bond group formed by a click reaction.
(7) The conjugate of any of (1) to (6), wherein the metal radionuclide is Ac-225, Y-90, Lu-177, or Zr-89.
(8) A conjugate of a chelating agent chelated with a radionuclide and a deglycosylated antibody, wherein the radionuclide is an α-ray emitting nuclide.
(9) The conjugate of (8) wherein the α-ray emitting nuclide is Ac-225.
(10) The conjugate of any one of (1) to (9), wherein the deglycosylated antibody is a human IgG antibody.
(11) The conjugate of any of (1) to (10), wherein the deglycosylation is performed by cleaving an N-type sugar chain.
(12) The conjugate of (11) wherein the N-type sugar chain is cleaved by treating with PNGaseF.
(13) A radiopharmaceutical comprising the conjugate of any of (1) to (12) as an active ingredient.
(14) The radiopharmaceutical of (13), which is used in an RI internal therapy for cancer.
(15) The radiopharmaceutical of (13), which is used in cancer diagnosis.
(16) A method for producing the conjugate of any of (1) to (12), comprising a conjugating step of producing a conjugate of a chelating agent chelated with a radionuclide and a deglycosylated antibody by conjugating the chelating agent and the antibody.
(17) The production method of (16), wherein the conjugating step is performed by a click reaction.
(18) The production method of (17), wherein the conjugating step is performed by a click reaction of the antibody with a radioactive metal complex of DOTAGA-DBCO represented by the following formula:

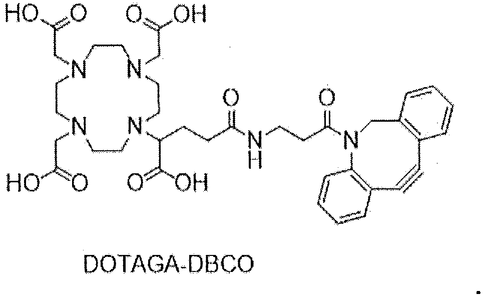

### [Example]

The present invention is described in more detail in the following by way of examples. However, the scope of the present invention is not limited by the examples.

### (Example 1] Production of deglycosylated antibody-RI conjugate by using ⁸⁹Zr-labeled DOTAGA-DBCO

### (1. Production of antibody composition) (step 1)

### (1-1. Production of antibody-modification linker)

A peptide (SEQ ID NO: 19) containing 17 amino acid residues represented by the following (P3) was obtained by the method described in WO 2017/217347. The amino acid sequence of this peptide was the same as the sequence in which Xaa2 of SEQ ID NO: 2 was a lysine residue, and the side chain terminal amino group of the lysine residue was modified with the structure shown by R₁. In addition, two cysteine residues form a disulfide bond with each other, and to the N-terminal of the peptide was added ethyl azide as an atomic group containing an azide group, which is the second atomic group, via a linker (L1) structure having diglycolic acid and eight PEGs.

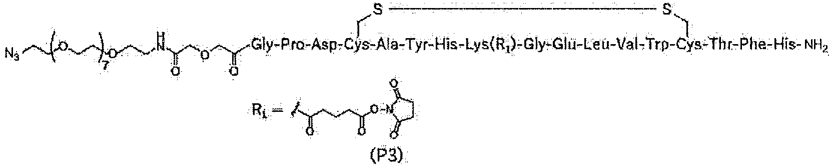

In the formula (P3), Lys is lysine, Gly is glycine, Pro is proline, Asp is aspartic acid, Cys is cysteine, Ala is alanine, Tyr is tyrosine, His is histidine, Glu is glutamic acid, Leu is leucine, Val is valine, Thr is threonine, Trp is tryptophan, and Phe is phenylalanine.

### (1-2. Modification of antibody with antibody-modification linker)

According to the method described in WO 2017/217347, the aforementioned peptide DSG (disuccinimidyl glutarate) was reacted. The obtained solution containing DSG-modified peptide and a solution containing the antibody deglycosylated with PNGaseF (Trastuzumab) which was obtained by the method described in J. Nucl. Med. 2019, 60, 1174 were mixed with a 0.02 mol/L acetic acid-sodium acetate buffer (pH 6.0) and the mixture was reacted at room temperature for 60 min to give a solution containing a peptide-modified antibody. The peptide-modified antibody had an Fc region of the antibody site-specifically modified by the above-mentioned peptide.

### (1-3. Separation and purification of monovalent antibody)

Then, the solution was passed through the IgG-BP column to obtain the first antibody composition containing relatively large amounts of the unlabeled antibody and monovalent antibody. The concentration of the monovalent antibody contained in the recovered fraction was adjusted with 0.02 mol/L phosphate buffer (pH 6.0) containing 0.1 mol/L sodium chloride such that the concentration was 15 mg/mL. An obtained solution containing the first antibody composition was subjected to the below-mentioned labeling step.

### (2. Complex formation step) (step 2)

DOTAGA-DBCO represented by the following formula was produced based on the method described in Chem. Eur. J. 2012, 18, 7834-7841. DOTAGA-DBCO was dispersed in 0.156 mol/L sodium acetate buffer (pH 5.5) as a solvent to give a dispersion containing 0.3 mmol/L chelating agent. A reaction mixture of the dispersion (0.03 mL), 0.156 mol/L sodium acetate buffer (pH 5.5, 0.030 mL) containing 0.15 mol/L gentinic acid, and ⁸⁹Zr ion-containing solution (⁸⁹Zr produced by ⁸⁹Y(p,n)⁸⁹Zr method and adjusted to 0.1 mol/L aqueous hydrochloric acid solution, radioactivity concentration 910 MBq/mL, liquid amount 0.03 mL) 27.3 MBq, as a radioactive metal source was reacted under heating conditions to give a ⁸⁹Zr complex solution. The molar ratio of the chelating agent and the radioactive metal ion was chelating agent:⁸⁹Zr ion = about 1500:1, and the heating temperature of the reaction mixture was set to 70°C, and the heating time was set to 1 hr.

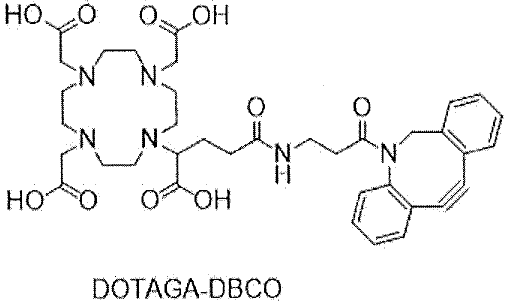

The radiochemical purity of the obtained ⁸⁹Zr complex was measured by the following method. That is, a part of the ⁸⁹Zr complex solution was developed by thin layer chromatography (manufactured by Agilent, model number: SGI0001, eluent = mixed solution of acetonitrile:0.1 mol/L EDTA solution (pH5.0) (volume ratio 1:1)), and then measured by radio-γ-TLC Analyzer (manufactured by raytest, MODEL GITA Star). The percentage of the radioactivity (count) of the peak detected near the point of origin with respect to the detected total radioactivity (count) was defined as the labeling rate (%) of the ⁸⁹Zr complex. As a result, the labeling rate of the ⁸⁹Zr complex was 96.0%. As used herein, the labeling rate (%) means the radioactivity of the ⁸⁹Zr complex relative to the charged radioactivity in the complex formation step. The obtained ⁸⁹Zr complex solution was directly used for the labeling step.

### (3. Labeling step) (step 3)

To a solution of the unpurified ⁸⁹Zr complex obtained via the aforementioned step 2 was added a solution containing a peptide-modified antibody (monovalent antibody) obtained in the above-mentioned step 1, and a click reaction was performed at 37°C for 2 hr to give ⁸⁹Zr complex-labeled antibody. The molar ratio of the DBCO group and the azide group at reaction was about 1:2.4. The reaction rate (%) of the unpurified ⁸⁹Zr complex-labeled antibody was 89.9%. Here, the reaction rate (%) means the radiochemical purity (RCP) (%) of the ⁸⁹Zr complex-labeled antibody with respect to the labeling rate (%) in the complex formation step.

Furthermore, a solution of the ⁸⁹Zr complex-labeled antibody obtained by reacting at 37°C for 2 hr was purified using ultrafiltration filter (manufactured by Merck, model number: UFC505096). The RCP of the ⁸⁹Zr complex-labeled antibody after purification was 96.5% and the radiochemical yield (RCY) thereof was 66.7%.

### (4. Formulation step) (step 4)

A portion of each of ⁸⁹Zr complex-labeled antibody produced in the above-mentioned step 3 was placed in a 5 mL Eppendorf tube (LoBind, manufactured by Eppendorf) and diluted with a preservation buffer (84 mg/L PS20-containig 19 g/L trehalose hydrate, 470 mg/L L-histidine hydrochloride, 300 mg/L L-histidine aqueous solution).

### [Comparative Example 1] Production of conjugate with antibody (not deglycosylated) using ⁸⁹Zr-labeled DOTAGA-DBCO

### (1. Antibody preparation step) (step 1)

As antibody, undeglycosylated Trastuzumab was used. The antibody-modification linker used was the same as that described in Example 1, and separation and purification of monovalent antibody was performed in the same manner as in Example 1.

### (2. Complex formation step) (step 2)

DOTAGA-DBCO was dispersed in 0.156 mol/L sodium acetate buffer (pH 5.5) as a solvent to give a dispersion containing 0.3 mmol/L chelating agent. A reaction mixture of the dispersion (0.03 mL), 0.156 mol/L sodium acetate buffer (pH 5.5, 0.030 mL) containing 0.15 mol/L gentinic acid, and ⁸⁹Zr ion-containing solution (⁸⁹Zr produced by ⁸⁹Y(p,n)⁸⁹Zr method and adjusted to 0.1 mol/L aqueous hydrochloric acid solution, radioactivity concentration 890 MBq/mL, liquid amount 0.03 mL) 26.7 MBq, as a radioactive metal source was reacted under heating conditions to give a ⁸⁹Zr complex solution. The molar ratio of the chelating agent and the radioactive metal ion was chelating agent:⁸⁹Zr ion = about 1500:1, and the heating temperature of the reaction mixture was set to 70°C, and the heating time was set to 1 hr.

The radiochemical purity of the obtained ⁸⁹Zr complex was measured in the same manner as in Example 1. As a result, the labeling rate of the ⁸⁹Zr complex was 97.7%. The obtained ⁸⁹Zr complex solution was directly used for the labeling step.

### (3. Labeling step) (step 3)

To a solution of the unpurified ⁸⁹Zr complex obtained via the aforementioned step 2 was added a solution containing a peptide-modified antibody (monovalent antibody) obtained in the same manner as in Example 1, and a click reaction was performed at 37°C for 2 hr to give ⁸⁹Zr complex-labeled antibody. The molar ratio of DBCO and azide at reaction was about 1:2.4. The reaction rate (%) of the unpurified ⁸⁹Zr complex-labeled antibody was 88.9%.

Furthermore, a solution of the ⁸⁹Zr complex-labeled antibody obtained by reacting at 37°C for 2 hr was purified using ultrafiltration filter (manufactured by Merck, model number: UFC505096). The RCP of the ⁸⁹Zr complex-labeled antibody after purification was 97.1% and RCY was 73%. The RCP and RCY of the ⁸⁹Zr complex-labeled antibody were measured in the same manner as in Example 1.

### (4. Formulation step) (step 4)

A portion of each of ⁸⁹Zr complex-labeled antibody produced in the above-mentioned step 3 was placed in a 5 mL Eppendorf tube (LoBind, manufactured by Eppendorf) and diluted with a preservation buffer (84 mg/L PS20-dontaining 19 g/L trehalose hydrate, 470 mg/L L-histidine hydrochloride, 300 mg/L L-histidine aqueous solution).

### [Evaluation 1] Proportion of aggregates

The proportion of aggregates contained in the ⁸⁹Zr complex-labeled antibody was confirmed by size-exclusion chromatography (SEC). Using Model 2695 Separation Module or e2695 Separation Module, manufactured by Water, as a liquid chromatography device, and Model 2489 UV/Vis Detector manufactured by Waters as a UV detector, analysis was performed under the following conditions. The proportion of each component after completion of the production is shown in Table 1.

**[Table 1]**

| evaluated compound | proportion of main peak (%) | proportion of aggregate peak (%) |
|---|---|---|
| ⁸⁹Zr complex-labeled deglycosylated antibody | 92.12 | 0.36 |
| ⁸⁹Zr complex-labeled antibody | 92.81 | 0.41 |

### [HPLC conditions]

column: TOSOH TSKgel guard column SWXL (6 mm×4 cm), TOSOH
TSKgel G3000SWXL (5 µm, 7.8×30 cm)×2 (tandem)
column temperature: constant temperature around 25°C
mobile phase: 0.2 mol/L arginine hydrochloride-containing 0.1
mol/L phosphate buffer (pH 6.8)
flow: 1.0 mL/min
area measurement range: 30 min
detection wavelength: 280 nm

### [Evaluation 2] Antigen binding activity

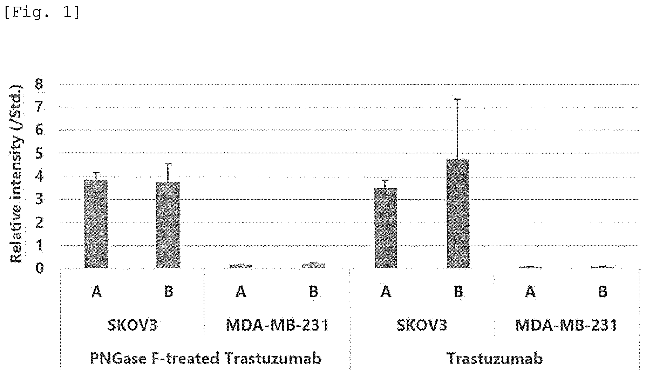

The antigen binding activity was confirmed by in vitro autoradiography (ARG). SK-OV-3 cells of HER2-positive human ovarian cancer cell line and MDA-MB-231 cells of HER2-negative human breast cancer cell line, which were purchased from ATCC (American Type Culture Collection), were administered subcutaneously to the flanks of female SCID Beige mice (Charles River Laboratories Japan, Inc.) each at 5×10⁶ cells to prepare tumor-bearing mice. Thereafter, SKOV3 tumor and MDA-MB-231 tumor were excised and embedded in Tissue-Tek O.C.T. Compound (Sakura Finetek Japan Co., Ltd.) to prepare frozen sections. The radioconjugates obtained in Example 1 and Comparative Example 1 were added to 1% bovine serum albumin-containing PBS each at 5 kBq/mL, and SKOV3 tumor section and MDA-MB-231 tumor section were immersed therein. After contacting the sections with the imaging plate, they were read with a scanner-type image analyzer (GE Healthcare Japan Corporation, Typhoon FLA 7000) to evaluate the level of radioactivity bound to the sections. The results are shown in Fig. 1. The vertical axis in the Figure indicates values obtained by setting any region of interest (ROI) in a tissue, quantifying intensity per unit area thereof, and dividing same by the intensity of the standard radiation source Std. (standard radiation source: 5 µL spot of solution containing the radioconjugate in which the section was immersed). Ten ROIs were set in the tissue, and the standard deviations thereof were taken.

The binding activity to HER2 was confirmed in all of the radioconjugates prepared as described in Example 1 and Comparative Example 1. Both radioconjugates prepared as described in Example 1 and Comparative Example 1 bound more strongly to HER2-positive SK-OV-3 tumor section and did not bind to HER2-negative MDA-MB-231 tumor section, demonstrating HER2 selective binding.

On the other hand, there was no difference in the binding ability to HER2 between antibodies with and without cleaved sugar chains.

### [Evaluation 3] Evaluation of distribution in the body

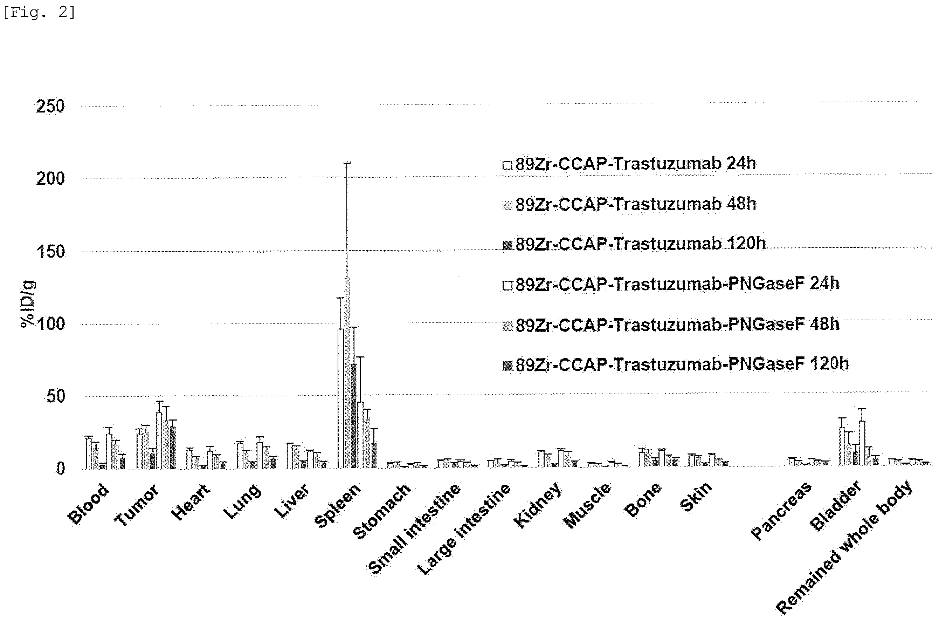

A subcutaneous tumor-bearing model of SKOV3 cells was prepared using mice, and the distribution of the radioconjugate produced as described in Example 1 and Comparative Example 1 to each tissue in the body was confirmed. SKOV3 cells were suspended in PBS containing 50% Matrigel, and subcutaneously transplanted to the left inguinal region of 5-week-old female NSG (official lineage name: NOD.Cg-Prkdc^{scid}I12rg^{tm1Wjl/}SzJ) mice (Charles River Laboratories Japan, Inc.) at 5×10⁶ cells to prepare tumor-bearing mice. The tumor-bearing mice were bred until the tumor volume reached about 50 to 200 mm³. The radioconjugates prepared as described in Example 1 or Comparative Example 1 were administered into the tail vein at a dose of 0.5 to 0.6 MBq/mouse (each n=3). After 24, 48, and 120 hr from the administration of radioconjugates, blood samples were collected from the heart under isoflurane anesthesia, the heart, lung, liver, spleen, pancreas, stomach, small intestine, large intestine, kidney, bladder, thigh muscle, femur, skin, and tumor were collected, and tissue other than those were collected as remained whole body. Mice administered with each radioconjugate were bred in metabolic cages until the time point of anatomy, and feces and urine were collected. The weights of the collected each tissue and blood were measured, and the counting rates of each tissue, the blood, feces, and urine were measured using a γ-ray scintillation measuring device (JDC-1712, manufactured by Hitachi, Ltd.). The accumulation rates (%ID/g) per unit weight was calculated from the obtained tissue weight and counting rate. The results are shown in Fig. 2.

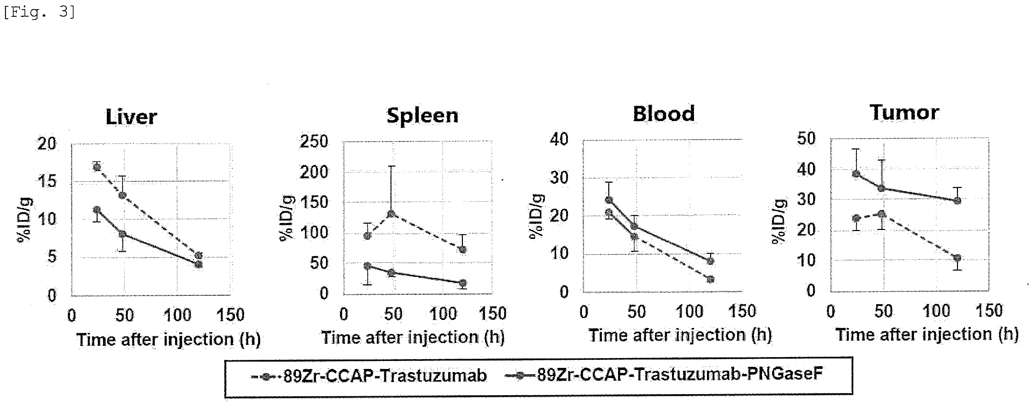

The ⁸⁹Zr-labeled deglycosylated antibody produced according to Example 1 showed low accumulation amounts at any time point in the liver and spleen, which are reticuloendothelial tissues, as compared with the ⁸⁹Zr-labeled sugar chain untreated antibody (undeglycosylated antibody) produced according to the description of Comparative Example 1 (Fig. 3). Furthermore, the amount of antibody retained in the blood was higher for the ⁸⁹Zr-labeled deglycosylated antibody than for the ⁸⁹Zr-labeled sugar chain untreated antibody at all time points, which results from the higher tumor accumulation rate for the ⁸⁹Zr-labeled deglycosylated antibody.

### [Example 2] Production of deglycosylated antibody-RI conjugate by using ²²⁵Ac-labeled DOTAGA-DBCO

### (1. Deglycosylated antibody preparation step) (step 1)

Using the deglycosylated antibody and the antibody-modification linker described in Example 1, the antibody was modified with the antibody-modification linker. Furthermore, a solution containing the first antibody composition obtained by the separation and purification method of monovalent antibody described in Example 1 was subjected to the below-mentioned labeling step.

### (2. Complex formation step) (step 2)

DOTAGA-DBCO was dispersed in 0.156 mol/L sodium acetate buffer (pH 5.5) as a solvent to give a dispersion containing 0.3 mmol/L chelating agent. A reaction mixture of the dispersion (0.080 mL), 0.2 mol/L aqueous hydrochloric acid solution (0.034 mL), and ²²⁵Ac ion-containing solution (0.2 mol/L aqueous hydrochloric acid solution, radioactivity concentration 157 MBq/mL, prepared from one produced by ROSATOM, liquid amount 0.006 mL) 0.94 MBq as a radioactive metal source was reacted under heating conditions to give a ²²⁵Ac complex solution. The molar ratio of the chelating agent and the metal radionuclide ion at that time was chelating agent:²²⁵Ac ion = about 12304:1, and the heating condition of the reaction mixture was set to 70°C, heating time 30 min.

The radiochemical purity of the obtained ²²⁵Ac complex was measured in the same manner as in Example 1. As a result, the labeling rate of the ²²⁵Ac complex was 81%. 0.04 mL (300 kBq, as ligand 8 nmol) from the obtained ²²⁵Ac complex solution was used for the labeling step.

### (3. Labeling step) (step 3)

To a solution of the unpurified ²²⁵Ac complex obtained via the aforementioned step 2 was added a solution (0.117 mL) containing a peptide-modified antibody (monovalent antibody) obtained in the above-mentioned step 1, and a click reaction was performed at 37°C for 1.5 hr to give ²²⁵Ac complex-labeled antibody. The molar ratio of the DBCO group and the azide group at reaction was about 1:1.5. The reaction rate of the unpurified ²²⁵Ac complex-labeled antibody was 77.6%.

Furthermore, a solution of the obtained ²²⁵Ac complex-labeled antibody was purified using ultrafiltration filter (manufactured by Merck, model number: UFC505096). The RCP of the ²²⁵Ac complex-labeled antibody after purification was 99.4% and RCY thereof was 62.0%. The RCP and RCY of the ²²⁵Ac complex-labeled antibody were measured in the same manner as in Example 1.

### (4. Formulation step) (step 4)

The ²²⁵Ac complex-labeled deglycosylated antibody produced in the above-mentioned step 3 was diluted with a preservation buffer in the same manner as in Example 1.

### [Comparative Example 2] Production of conjugate with antibody (not deglycosylated) by using ²²⁵Ac-labeled DOTAGA-DBCO

### (1. Antibody preparation step) (step 1)

Using the undeglycosylated antibody and the antibody-modification linker described in Comparative Example 1, the antibody was modified with the antibody-modification linker. Furthermore, a solution containing the first antibody composition obtained by the separation and purification method of monovalent antibody described in Example 1 was subjected to the below-mentioned labeling step.

### (2. Complex formation step) (step 2)

DOTAGA-DBCO was dispersed in 0.156 mol/L sodium acetate buffer (pH 5.5) as a solvent to give a dispersion containing 0.3 mmol/L chelating agent. A reaction mixture of the dispersion (0.080 mL), 0.2 mol/L aqueous hydrochloric acid solution (0.034 mL), and ²²⁵Ac ion-containing solution (0.2 mol/L aqueous hydrochloric acid solution, radioactivity concentration 157 MBq/mL, prepared from one produced by ROSATOM, liquid amount 0.006 mL) 0.94 MBq as a radioactive metal source was reacted under heating conditions to give a ²²⁵Ac complex solution. The molar ratio of the chelating agent and the metal radionuclide ion at that time was chelating agent:²²⁵Ac ion = about 12304:1, and the heating condition of the reaction mixture was set to 70°C, heating time 30 min.

The RCP of the obtained ²²⁵Ac complex was measured in the same manner as in Example 1. As a result, the RCP of the ²²⁵Ac complex was 81%. 0.04 mL (307 kBq, as ligand 8 nmol) from the obtained ²²⁵Ac complex solution was used for the labeling step.

### (3. Labeling step) (step 3)

To a solution of the unpurified ²²⁵Ac complex obtained via the aforementioned step 2 was added a solution (0.137 mL) containing the peptide-modified antibody (monovalent antibody) obtained in step 1, and a click reaction was performed at 37°C for 1 hr to give a ²²⁵Ac complex-labeled antibody. The molar ratio of DBCO and azide at reaction was each about 1:1.7. The reaction rate of the unpurified ²²⁵Ac complex-labeled antibody was 79.8%.

Furthermore, a solution of the ²²⁵Ac complex-labeled antibody obtained by reacting at 37°C for 2 hr was purified using ultrafiltration filter (manufactured by Merck, model number: UFC505096). The RCP of the ²²⁵Ac complex-labeled antibody after purification was 99.7% and the RCY thereof was 60.9%. The RCP and RCY of the ²²⁵Ac complex-labeled antibody were measured in the same manner as in Example 1.

### (4. Formulation step) (step 4)

The ²²⁵Ac complex-labeled antibody produced in the above-mentioned step 3 was diluted with a preservation buffer in the same manner as in Comparative Example 1.

### [Evaluation 4] Evaluation of aggregate

Whether the ²²⁵Ac complex-labeled antibody contained aggregates was confirmed by dynamic light scattering (DLS). Analysis was performed under the following conditions using DynaPro NanoStar (manufacturing number: 693-DPN) manufactured by WYATT as a DLS measuring device. The average particle size after production was 10.6 nm for the ²²⁵Ac complex-labeled deglycosylated antibody and 9.2 nm for the ²²⁵Ac complex-labeled sugar chain-untreated antibody. Since all particles were within the range of 9 to 12 nm, which is the average particle size of antibodies, no progress of aggregation or the like was suggested.

### [DLS measurement conditions]

measurement time: 5 seconds
measurement interval: 1 second
number of measurements: 10 times×3 times
measurement temperature: 25°C
laser wavelength: 658 nm
laser output: 10 to 100 mW (variable)

### [Evaluation 5] Antigen binding activity

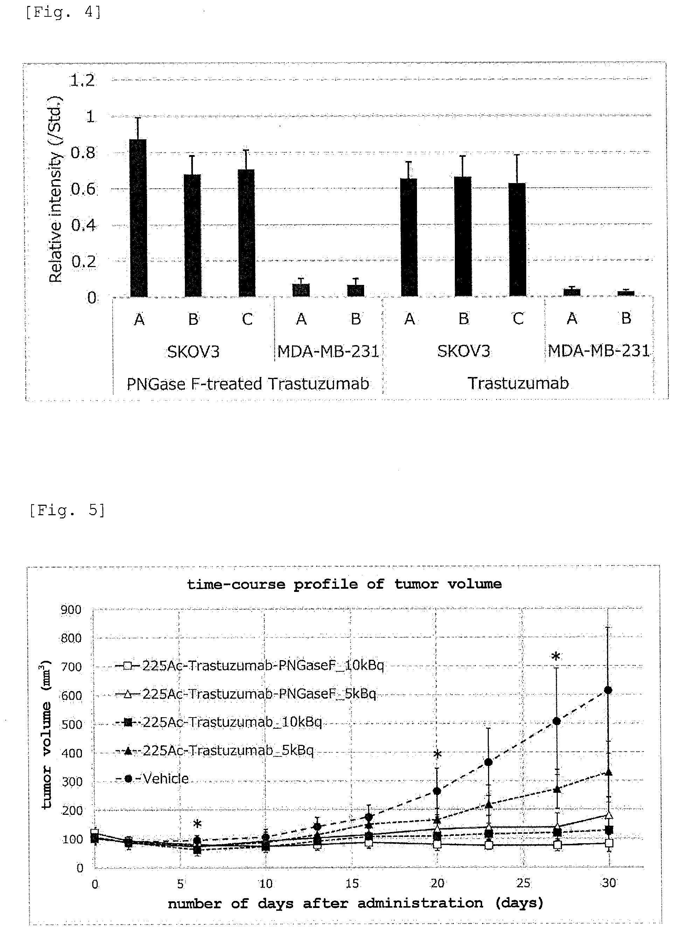

By a method similar to that described in Evaluation 2, frozen sections of SKOV3 tumor and MDA-MB-231 tumor were prepared. The radioconjugates obtained in Example 2 and Comparative Example 2 were added to 1% bovine serum albumin-containing PBS each at 1 kBq/mL, and SKOV3 tumor section and MDA-MB-231 tumor section were immersed therein. The level of radioactivity bound to the sections was evaluated by a method similar to that described in Evaluation 2 and the results are shown in Fig. 4.

The radioconjugates prepared as described in Example 2 and Comparative Example 2 bound more strongly to HER2-positive SKOV3 tumor section and did not bind to HER2-negative MDA-MB-231 tumor section, demonstrating HER2 selective binding. On the other hand, there was no difference in the binding ability to HER2 between antibodies with and without cleaved sugar chains.

### [Evaluation 6] Efficacy evaluation using ²²⁵Ac complex-labeled antibody

Subcutaneous tumor-bearing model of SKOV3 was prepared using mice, and the antitumor effect of the radioconjugates prepared as described in Example 2 and Comparative Example 2 was confirmed.

SKOV3 cells were suspended in McCoy's 5A medium (Gibco) containing 50% Matrigel and administered subcutaneously to the right shoulder of 5-week-old female NSG mice (manufactured by The Jackson Laboratory Japan, Inc.) at 5×10⁶ cells to prepare tumor-bearing mice. The mice were bred until the tumor volume reached about 50 to 200 mm³, and individuals with a shape suitable for tumor diameter measurement were randomly grouped. The tumor volume and body weight of each mouse at that time are shown in Table 2. The tumor volume was calculated according to the following formula. tumor volume (mm3) = (tumor major axis x (tumor minor axis) 2) x 1/2

**[Table 2]**

| | tumor volume mean±standard deviation (mm³) | body weight mean±standard deviation (g) |
|---|---|---|
| radioconjugate (Example 2) | 121.6±19.1 | 18.9±1.0 |
| 10 kBq administration group | | |
| radioconjugate (Example 2) | 106.0±26.4 | 18.9±1.1 |
| 5 kBq administration group | | |
| radioconjugate (Comparative Example 2) | 107.2±11.6 | 18.8±1.3 |
| 10 kBq administration group | | |
| radioconjugate (Comparative Example 2) | 101.7±17.4 | 18.6±1.1 |
| 5 kBq administration group | | |
| Vehicle group | 102.4112.1 | 18.8±1.4 |

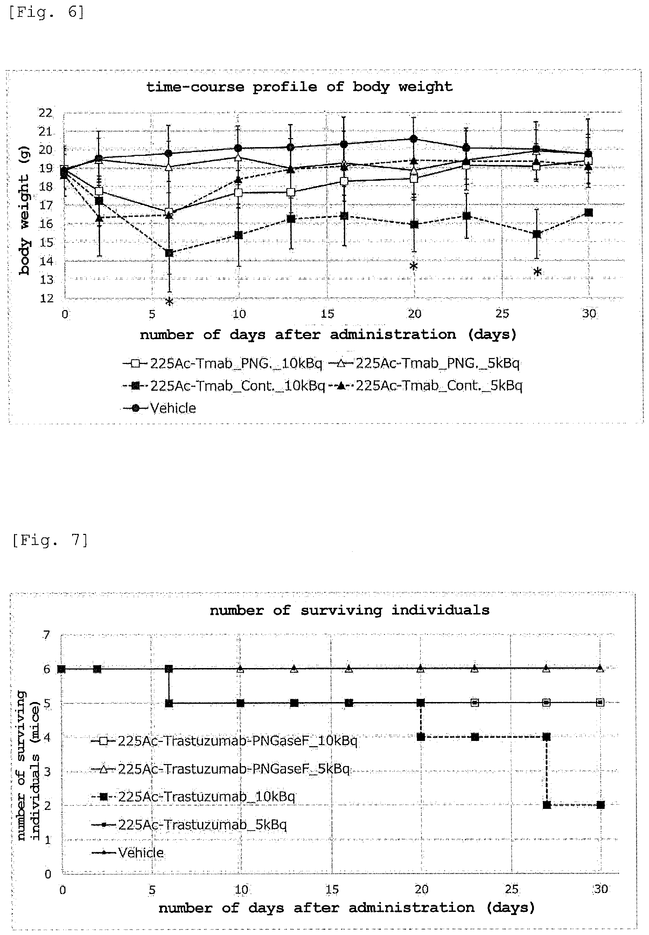

²²⁵Ac complex-labeled antibodies were administered into the tail vein at a dose of 10 kBq or 5 kBq/mouse (about 100 µg/mouse and 50 µg/mouse as Trastuzumab, respectively). As a control group, a Vehicle group administered with a preservation buffer were set. Each group contained 6 mice, and observation of general condition and measurement of the body weight and tumor volume were performed over time for 30 days after administration. Mice whose body weight was less than 80% of the body weight on the day of administration and whose physical activity and eating behavior were abnormal were euthanized by applying humane endpoints. Time-course changes in tumor volume are shown in Fig. 5, time-course changes in body weight change are shown in Fig. 6, and the number of surviving individuals during the observation period is shown in Fig. 7. The vertical axis of the graph in Fig. 5 represents the tumor volume, and the horizontal axis represents the number of days that have passed since administration of each medicament. The graph is expressed as the mean±standard deviation of tumor volume in each group. The vertical axis of the graph in Fig. 6 represents the weight of the mouse, and the horizontal axis represents the number of days that have passed since administration of each medicament. The vertical axis of the graph in Fig. 7 shows the number of surviving individuals, and the horizontal axis shows the number of days that have passed since administration of each medicament. In the Figure, "*" indicates the point at which the individual was euthanized by applying humane endpoints.

At 30 days after administration, a difference in antitumor effect was observed in the group to which the ²²⁵Ac complex-labeled antibody was administered as compared with the Vehicle group. Furthermore, in both the 10 kBq administration group and the 5 kBq administration group, the average tumor volume of the group administered with the antibody produced in Example 2 was smaller than that of the antibody produced in Comparative Example 2. Therefore, it was suggested that deglycosylated antibodies exhibit stronger antitumor effects.

In addition, in the group to which the antibody produced in Example 2 was administered, a total of one animal was subjected to humane endpoints due to a decrease in body weight or physical activity, whereas in the group administered with the antibody produced in Comparative Example 2, a total of 5 animals were subjected to the humane endpoints. These results suggest that deglycosylation of antibodies can reduce side effects such as decrease in body weight and physical activity.

### [Example 3] Production of deglycosylated antibody-RI conjugate using ⁸⁹Zr-labeled DOTAGA-DBCO -2

### (1. Antibody preparation step) (step 1)

Deglycosylated Cetuximab was used as the antibody. The antibody modification linker used was the same as that described in Example 1, and the monovalent antibody was separated and purified in the same manner as in Example 1.

### (2. Complex formation step) (step 2)

Performed in the same manner as in Example 1 except that the radioactivity during complex formation was set to 87.7 MBq.

RCP of the obtained ⁸⁹Zr complex was measured in the same manner as in Example 1. As a result, RCP of ⁸⁹Zr complex was 97.8%. 0.03 mL (25.9 MBq, as ligand 3 nmol) from the obtained ⁸⁹Zr complex solution was used for a labeling step.

### (3. Labeling step) (step 3)

Performed in the same manner as in Example 1 except that deglycosylated Cetuximab was used as the antibody, and the molar ratio of DBCO and azide during the reaction was set to about 1:1.2. The reaction rate of the unpurified ⁸⁹Zr complex-labeled antibody was 56.1%.

Furthermore, a solution of the obtained ⁸⁹Zr complex-labeled antibody was purified using ultrafiltration filter (manufactured by Merck, model number: UFC505096). The RCP of the ⁸⁹Zr complex-labeled antibody after purification was 93.9% and the RCY thereof was 46.6%. The RCP and RCY of the ⁸⁹Zr complex-labeled antibody were measured in the same manner as in Example 1.

### (4. Formulation step) (step 4)

A portion of each of ⁸⁹Zr complex-labeled deglycosylated antibody produced in the above-mentioned step 3 was placed in a 5 mL Eppendorf tube (LoBind, manufactured by Eppendorf) and diluted with a preservation buffer (100 mg/L PS20-containing 5844 mg/L sodium chloride, 7507 mg/L glycine, 2101 mg/L citric acid hydrate aqueous solution).

### [Comparative Example 3] Production of conjugate with antibody (not deglycosylated) using ⁸⁹Zr-labeled DOTAGA-DBCO -2

### (1. Antibody preparation step) (step 1)

As antibody, undeglycosylated Cetuximab was used. The antibody-modification linker used was the same as that described in Example 1, and separation and purification of monovalent antibody was performed in the same manner as in Example 1.

### (2. Complex formation step) (step 2)

Performed in the same manner as in Example 3.

The RCP of the obtained ⁸⁹Zr complex was measured in the same manner as in Example 1. As a result, the RCP of the ⁸⁹Zr complex was 97.8%. 0.03 mL (28.7 MBq, as ligand 3 nmol) from the obtained ⁸⁹Zr complex solution was used for the labeling step.

### (3. Labeling step) (step 3)

Performed in the same manner as in Example 1 except that undeglycosylated Cetuximab was used as the antibody, and the molar ratio of DBCO and azide during the reaction was set to about 1:1.2. The reaction rate of the unpurified ⁸⁹Zr complex-labeled antibody was 51.4%.

Furthermore, a solution of the obtained ⁸⁹Zr complex-labeled antibody was purified using ultrafiltration filter (manufactured by Merck, model number: UFC505096). The RCP of the ⁸⁹Zr complex-labeled antibody after purification was 93.8% and RCY was 50.7%. The RCP and RCY of the ⁸⁹Zr complex-labeled antibody were measured in the same manner as in Example 1.

### (4. Formulated step) (step 4)

A portion of ⁸⁹Zr complex-labeled antibody of the above-mentioned step 3 (not deglycosylated) was placed in a 5 mL Eppendorf tube (LoBind, manufactured by Eppendorf) and diluted with a preservation buffer (100 mg/L PS20-containing 5844 mg/L sodium chloride, 7507 mg/L glycine, 2101 mg/L citric acid hydrate aqueous solution).

### [Evaluation 7] Proportion of aggregates

The proportion of aggregates contained in the ⁸⁹Zr complex-labeled antibodies produced in Example 3 and Comparative Example 3 was analyzed by SEC in the same manner as in Evaluation 1. HPLC conditions were similarly set as in Evaluation 1. The proportion of each component after completion of the production is shown in Table 3.

**[Table 3]**

| evaluated compound | proportion of main peak (%) | proportion of aggregate peak (%) |
|---|---|---|
| ⁸⁹Zr complex-labeled deglycosylated antibody (Example 3) | 98.46 | 1.54 |
| ⁸⁹Zr complex-labeled antibody (Comparative Example 3) | 99.26 | 0.74 |

### [Evaluation 8] Antigen binding activity

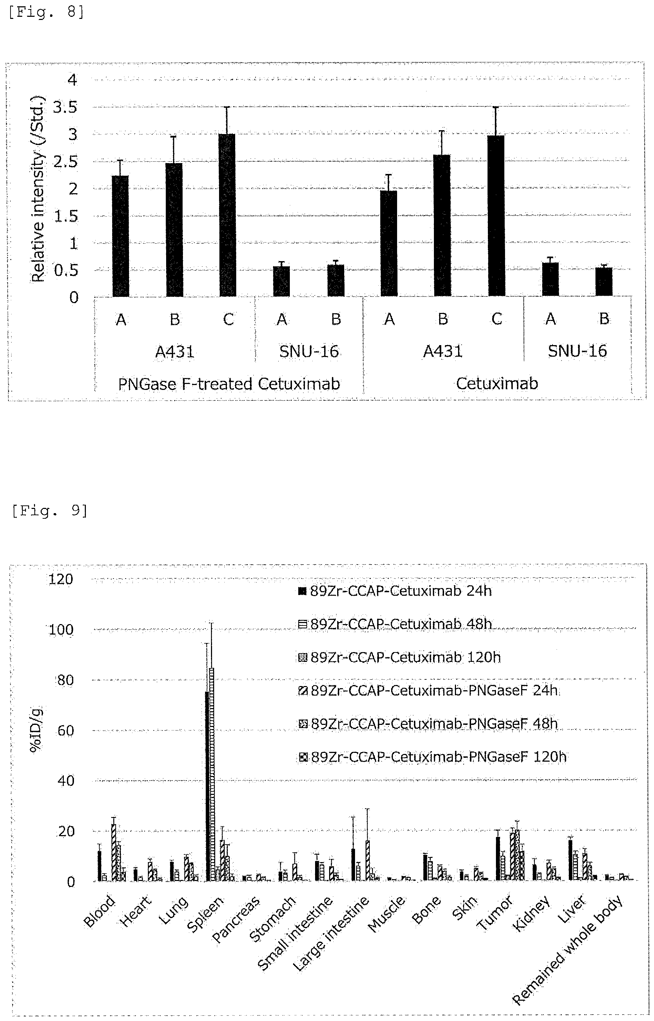

By a method similar to that described in Evaluation 2, tumor frozen sections were produced. However, the A431 section prepared using NSG mice was used as the positive section, and the SNU-16 section, which is a low expression section prepared using SCID Beige mice, was used as a substitute for the negative section. The results are shown in Fig. 8.

The binding activity to EGFR was confirmed in all radioconjugates produced according to the descriptions of Example 3 and Comparative Example 3. The ⁸⁹Zr complex-labeled antibody bound more strongly to EGFR-positive A431 tumor sections, and showed lower bindability to SNU-16 tumor sections, which have low EGFR expression, thus indicating EGFR-selective binding. On the other hand, no difference was found in the binding ability to EGFR between deglycosylated antibodies and sugar chain untreated antibodies.

### [Evaluation 9] Evaluation of distribution in the body

Similar to Evaluation 3, a subcutaneous tumor-transplanted model of A431 tumor was prepared using mice, and the distribution of the radioconjugate produced as described in Example 3 and Comparative Example 3 to each tissue in the body was confirmed. A431 cells were suspended in DMEM medium (manufactured by gibco), and subcutaneously transplanted to the right shoulder of 5-week-old female NSG mice (manufactured by The Jackson Laboratory Japan, Inc.) at 5×10⁶ cells to prepare tumor-bearing mice. The tumor-bearing mice were bred until the tumor volume reached about 50 to 200 mm³. ⁸⁹Zr complex-labeled antibody was administered into the tail vein at a dose of 0.5 to 0.6 MBq/mouse (each group n=4). After 24, 48, and 120 hr from the administration of radioconjugates, blood samples were collected from the heart under isoflurane anesthesia, the heart, lung, spleen, pancreas, stomach, small intestine, large intestine, thigh muscle, femur, skin, tumor, kidney, and liver were collected, and tissue other than those were collected as remained whole body. Mice administered with each radioconjugate were bred in metabolic cages until the time point of anatomy, and feces and urine were collected. The weights of the collected each tissue and blood were measured, and the counting rates of each tissue, the blood, feces, and urine were measured using a γ-ray scintillation measuring device (JDC-1712, manufactured by Hitachi, Ltd.). The accumulation rates (%ID/g) per unit weight was calculated from the obtained tissue weight and counting rate. The results are shown in Fig. 9. The vertical axis of the graph shows the accumulation rate per unit weight of each tissue.

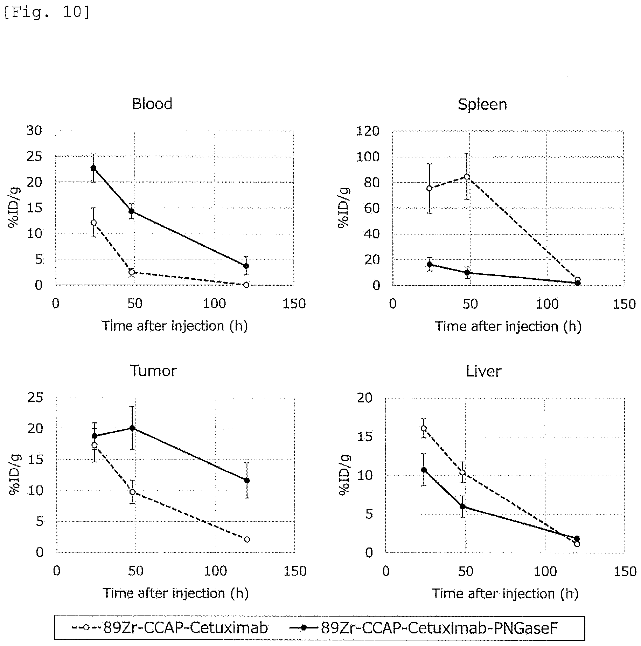

The ⁸⁹Zr-labeled deglycosylated antibody produced according to Example 3 showed low accumulation amounts in the liver and spleen, which are reticuloendothelial tissues, as compared with the ⁸⁹Zr-labeled sugar chain untreated antibody produced according to the description of Comparative Example 3 (Fig. 10). Furthermore, the amount of antibody retained in the blood was higher for the ⁸⁹Zr-labeled deglycosylated antibody than for the ⁸⁹Zr-labeled sugar chain untreated antibody at all time points, which leads to the higher tumor accumulation rate for the ⁸⁹Zr-labeled deglycosylated antibody.

This application is based on a patent application No. 2021-141625 filed in Japan (filing date: August 31, 2021), the contents of which are incorporated in full herein.

## Claims

1. A conjugate of a chelating agent chelated with a radionuclide and a deglycosylated antibody, wherein the chelating agent site-specifically modifies Fc region of the antibody via a linker.

2. The conjugate according to claim 1, wherein the linker comprises an antibody-modification peptide composed of not less than 13 and not more than 17 amino acid residues represented by the following formula (i):
(Xa)-Xaa1-(Xb)-Xaa2-(Xc)-Xaa3-(Xd)... (i)
wherein Xa, Xb, Xc and Xd are continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy a+b+c+d≤14,
Xaa1 and Xaa3 are each independently an amino acid residue derived from an amino acid having a thiol group in the side chain, and they are bonded via a disulfide bond or their sulfide groups are bonded via a linker, or
one is an amino acid residue derived from an amino acid having a thiol group in the side chain and the other is an amino acid residue derived from an amino acid having a haloacetyl group in the side chain, and they are bonded via a thioether bond, and
Xaa2 is a lysine residue, an arginine residue, a cysteine residue, an aspartic acid residue, a glutamic acid residue, 2-aminosuberic acid, or diamino propionic acid.

3. The conjugate according to claim 2, wherein the antibody-modification peptide is the formula (i) wherein Xaa2 is a lysine residue.

4. The conjugate according to claim 2, wherein the antibody-modification peptide comprises an antibody-modification peptide consisting of the amino acid sequence shown in SEQ ID NO: 2 (wherein Xaa2 is a lysine residue).

5. The conjugate according to claim 1 or 2, wherein the chelating agent has a structure derived from a compound represented by the following formula (A) or a salt thereof: 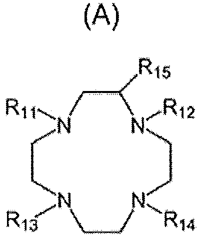 in the formula (A), R₁₁, R₁₃, and R₁₄ are each independently a group consisting of -(CH₂)ₚCOOH, - (CH₂)ₚC₅H₅N, -(CH₂)ₚPO₃H₂, - (CH₂)ₚCONH₂, or -(CHCOOH)(CH₂)ₚCOOH, one of R₁₂ and R₁₅ is a hydrogen atom, a carboxyl group, or a carboxyalkyl group having 2 or 3 carbon atoms, the other is a substituent for conjugating with the antibody, p is an integer of not less than 0 and not more than 3, when R₁₂ is a substituent for conjugating with the antibody, then R₁₅ is a hydrogen atom, and when R₁₂ is not a substituent for conjugating with the antibody, then R₁₅ is a substituent for conjugating with the antibody.

6. The conjugate according to claim 1 or 2, wherein the linker has a bond group formed by a click reaction.

7. The conjugate according to claim 1 or 2, wherein the radionuclide is Ac-225, Y-90, Lu-177, or Zr-89.

8. A conjugate of a chelating agent chelated with a radionuclide and a deglycosylated antibody, wherein the radionuclide is actinium-225 (Ac-225).

9. The conjugate according to claim 1 or 8, wherein the deglycosylated antibody is a human IgG antibody.

10. The conjugate according to claim 1 or 8, wherein the deglycosylation is performed by cleaving an N-type sugar chain.

11. A radiopharmaceutical comprising the conjugate according to claim 1 or 8 as an active ingredient.

12. The radiopharmaceutical according to claim 11, which is used in an RI internal therapy for cancer.

13. The radiopharmaceutical according to claim 11, which is used in cancer diagnosis.

14. A method for producing the conjugate according to claim 1 or 8, comprising a conjugating step of producing a conjugate of a chelating agent chelated with a radionuclide and a deglycosylated antibody by conjugating the chelating agent and the antibody.

15. The production method according to claim 14, wherein the conjugating step is performed by a click reaction.

16. The production method according to claim 15, wherein the conjugating step is performed by a click reaction of the antibody with a radioactive metal complex of DOTAGA-DBCO represented by the following formula:
